# EUROPÄISCHE PATENTSCHRIFT

(11) **EP 3 425 637 B1**
(45) Veröffentlichungstag und Bekanntmachung des Hinweises auf die Patenterteilung: **26.07.2023**
(21) Anmeldenummer: 18176402.8
(22) Anmeldetag: 07.06.2018
(51) Int. Cl.: G16H 80/00, G06F 21/32, G16H 50/70, G16H 50/20, G06F 21/31

(54) **ELEKTRONISCHES SYSTEM UND VERFAHREN ZUR KLASSIFIKATION EINES PHYSIOLOGISCHEN ZUSTANDES**
ELECTRONIC SYSTEM AND METHOD FOR CLASSIFYING A PHYSIOLOGICAL STATE
SYSTÈME ÉLECTRONIQUE ET MÉTHODE DE CLASSIFICATION D'UN ÉTAT PHYSIOLOGIQUE

(30) Priorität: 07.07.2017 DE 102017211631
(43) Veröffentlichungstag der Anmeldung: 09.01.2019
(73) Patentinhaber: Bundesdruckerei GmbH, 10969 Berlin (DE)
(72) Erfinder: PAESCHKE, Manfred, 16348 Wandlitz (DE); SCHNJAKIN, Maxim, 10245 Berlin (DE)
(74) Vertreter: Richardt Patentanwälte PartG mbB

(56) Entgegenhaltungen:
- US-A1- 2015 065 803
- US-A1- 2015 106 020
- US-A1- 2015 109 124
- US-A1- 2015 310 444
- US-A1- 2016 182 503
- US-A1- 2016 302 671
- US-A1- 2017 035 328
- US-A1- 2017 161 438
- US-A1- 2017 174 180
- Wikipedia: "Elektronischer Heilberufsausweis", , 15. Juni 2017 (2017-06-15), XP055535964, Gefunden im Internet: URL:https://de.wikipedia.org/w/index.php?t itle=Elektronischer_Heilberufsausweis&oldi d=166417082 [gefunden am 2018-12-18]

## Beschreibung

Die Erfindung betrifft ein elektronisches System und ein Verfahren zur Klassifikation eines physiologischen Zustandes eines Nutzers.

Smartgeräte, wie etwa Smartphones oder Smartwatches, werden in vielen alltäglichen Situationen für die verschiedensten Aufgaben des Alltags eingesetzt. Sie unterstützen den Menschen durch Ihre Handhabbarkeit in Verbindung mit ihrer Rechenleistung und ihrer Sensorik. Die Sensorik ist dabei sensibel genug, beispielsweise den Puls und/oder Blutdruck eines Nutzers zu messen oder die Schritte des Nutzers zu zählen.

Handelsübliche Fitnesstracker unterstützen den Nutzer bei seinen sportlichen Aktivitäten, indem sie die einzelnen Körperfunktionen überwachen und aufzeichnen. Der Nutzer kann sich dann nach dem Training die erfassten Daten ansehen und diese auswerten.

Viele mobile, tragbare Kommunikationssysteme, wie Smartphones oder Smartwatches, sind mit Sensoren ausgestattet, mit welchen sich biometrische Daten des Nutzers erfassen lassen. Drucksensoren erfassen an geeigneten Stellen, wie beispielsweise dem Handgelenk oder der Schläfe, den Puls und den Blutdruck, an einer weiteren Stelle wird die Körpertemperatur durch einen Temperatursensor erfasst. Die Kombination der aktuell erfassten biometrischen Daten repräsentiert dabei einen aktuellen physiologischen Zustand des Nutzers. Der dargestellte physiologische Zustand ist dabei bezüglich einer Mehrzahl von Parametern höchst individuell. Beispielsweise kann der Ruhepuls eines jeden Menschen unterschiedlich sein. Andere Parameter wiederum sollten im Allgemeinen für alle Individuen in einem bestimmten Bereich liegen. Die Körpertemperatur sollte beispielsweise bei jedem Menschen um die 37°C liegen.

Da das Bestimmen der Parameter jedoch Fehlern und Störgrößen unterliegt, beispielsweise Erschütterungen durch Bewegung im Fall von Drucksensoren oder Einflüsse der Umgebungstemperatur im Fall von Temperatursensoren, ist eine exakte Auswertung schwierig. Ferner kann ein Körper eines Nutzers sehr komplex darauf reagieren, dass sich ein einzelner physiologischer Parameter des Körpers ändert.

Der Erfindung liegt demgegenüber die Aufgabe zugrunde, ein verbessertes elektronisches System und ein Verfahren zur Klassifikation eines physiologischen Zustandes eines Nutzers zu ermöglichen.

Die US 2015/065803 A1 beschreibt Vorrichtungen und Verfahren zur mobilen Erfassung und Analyse von Bildern des Trommelfells, um Informationen bereitzustellen zum Verständnis und zur Behandlung von Krankheiten, wie Ohrenentzündungen. Diese Vorrichtungen leiten und lenken ein Subjekt beim Aufnehmen eines Bildes eines Trommelfells, einschließlich des automatischen Erfassens, in welche Richtung die Position der Vorrichtung ausgerichtet werden soll, um ein Bild des Trommelfells aufzunehmen, und des automatischen Anzeigens, wann das Trommelfell abgebildet worden ist.

Die US 2015/106020 A1 beschreibt eine drahtlose physiologische Fernüberwachung bei der Beurteilung eines Gesundheitszustands und Krankheitszustands. Personalisierte Schwellenwerte werden verwendet, um die Leistung von Klassifizierungs- und Vorhersagemethoden zu verbessern, indem eine klinische Geschichte des Patienten und vergangene empirisch erfasste Daten verwendet werden, um die in jedem erfassten Individuum vorhandene biologische Variation zu erlernen. Daten von einem anhaftenden Patch werden einer verteilten Verarbeitung unterzogen, wobei die Daten sicher von einem Erfassungsgerät an einen Remote-Server gesendet werden. Überwachungs-, Klassifizierungs- und Prognoseergebnisse werden gehostet und über ein authentifiziertes System für Mitglieder eines Gesundheitsteams, einer Familie oder anderer Pflegepersonen des Individuums zugänglich gemacht. Lernalgorithmen werden verwendet, um physiologische Attribute zu charakterisieren und einen Gesundheits- oder Krankheitszustand auf der Grundlage einer Gesamtheit von Merkmalen zu klassifizieren.

Die US 2017/161438 A1 beschreibt ein Erfassen eines Indikators auf einem Computergerät dafür, dass ein Insasse ein Fahrzeug bestiegen hat. Eine Identifizierung des Insassen basiert auf dem Hinweis dafür, dass der Insasse in das Fahrzeug bestiegen hat. Vom dem Computergerät wird auf eine Gesundheitsakte zugegriffen, die dem Insassen zugeordnet ist. Das Computergerät ermöglicht es, dass die dem Insassen zugeordnete Gesundheitsakte, auf die zugegriffen wird, aus einem sicheren digitalen Speicherbehälter verfügbar ist. Die dem Insassen zugeordnete Gesundheitsakte wird einem zweiten Computergerät, das für einen Ersthelfer registriert ist, aus dem sicheren digitalen Speicherbehälter bereitgestellt basierend auf der Erfassung eines Indikators dafür, dass das Fahrzeug eine Kollision hatte.

Der Artikel "Elektronischer Heilberufsausweis" in der Wikipedia vom 15. Juni 2017 (URL:https://de.wikipedia.org/w/index.php?title=Elektronischer Heilberufsausweis&oldid=166417082) beschreibt einen Elektronische Heilberufsausweis (eHBA), bei welchem es sich um einen personenbezogenen Sichtausweis im Scheckkarten-Format handelt, welcher als Ausweis für die Gesundheitsberufe spezifiziert und die Grundlage von elektronischen Ausweisen im Arzt-, Apotheken-, Zahnarzt- und Psychotherapeutenbereich ist.

Die US 2015/310444 A1 beschreibt ein adaptives biometrisches Authentifizierungssystem, welches ein Nutzeridentitätsreferenzmodul umfasst, das dazu konfiguriert ist, in einem sicheren Speicher gespeicherte Nutzeridentifikationselemente zu verwalten. Das System umfasst einen passiven Datenaggregator, der konfiguriert ist, Datenelemente zu empfangen und zu aggregieren, die passiv von einem Gerät gesammelt werden, das sich in der Nähe eines Nutzers befindet. Das System enthält ferner einen Nutzeridentitätsvertrauensniveau-Generator, der dazu konfiguriert ist, ein Nutzeridentitätsvertrauensniveau zu erzeugen, welches ein Vertrauen dafür angibt, dass der Nutzer in der Nähe des Geräts der Referenznutzer ist, zumindest teilweise basierend auf einem Vergleich zwischen den passiv gesammelten Datenelementen und den Nutzeridentifikationselementen, sowie dazu, das Nutzeridentitätsvertrauensniveau zu aktualisieren, wenn zusätzliche passiv gesammelte Datenelemente empfangen werden. Das System umfasst ein Authentifizierungsmodul, welches die Nutzerauthentifizierung zumindest teilweise auf der Basis des Vertrauensniveaus der Nutzeridentität ermöglicht.

Die US 2015/109124 A1 beschreibt ein Verfahren, welches ein Verarbeiten von Daten in einem Datensatz umfasst, der zeitveränderliche Informationen über mindestens eine sich durch Blut in einem Subjekt ausbreitende Pulsdruckwelle darstellt, welche an einem Ort des Subjekts erfasst werden. Das Verfahren umfasst ferner ein Bestimmen, ob ein oder mehrere Segmente des Datensatzes von einem anderen Subjekt als einem erwarteten Subjekt erfasst wurden, indem morphologische Merkmale der Segmente analysiert werden.

Die US 2016/182503 A1 beschreibt eine Technologie zum Ausführen einer kontinuierlichen Authentifizierung eines mobilen Geräts, welche Nutzeraktivitätskontextdaten und biometrische Signaturdaten, die sich auf den Nutzer beziehen, verwendet. Eine biometrische Signatur wird basierend auf dem Aktivitätskontext ausgewählt, und die ausgewählte biometrische Signatur wird verwendet, um die Identität des Nutzers zu überprüfen.

Die US 2017/174180 A1 beschreibt Systeme und Verfahren zum Autorisieren eines Nutzers zum Zugreifen auf eine zugriffsgesteuerte Umgebung. Das System umfasst eine Systemserverplattform, die mit Mobilgeräten und Fahrzeugcomputern kommuniziert, auf die Nutzer zugreifen. Eine Reihe von Operationen wird ermöglicht, bei denen ein Nutzer, der auf ein Fahrzeug zugreift, aufgefordert wird, sich unter Verwendung des Mobilgerätes des Nutzers oder des Fahrzeugcomputers biometrisch zu authentifizieren. Zusätzlich autorisiert das System den Nutzer, erleichtert elektronisch den Zugang zum Fahrzeug und führt andere autorisierte Operationen in Bezug auf die Verwendung des Fahrzeugs aus. Darüber hinaus ist das Fahrzeugzugangssystem in verschiedene Computergeräte und computerbasierte Dienste integriert, auf die der Nutzer zugreifen kann. Die Systeme und Verfahren erleichtern ferner eine aktive Überwachung der Fahrzeuginsassen und der Umgebungsbedingungen unter Verwendung von optischen Sensoren, um die Sicherheit, den Komfort und den Schutz der Insassen während der Benutzung des Fahrzeugs zu verbessern.

Die US 2016/0302671 A1 beschreibt ein Verfahren zum Sammeln und Analysieren physiologischer Informationen zum Vorhersagen, wann eine Person wahrscheinlich Fieber entwickelt, bevor die Körpertemperatur dieser Person ansteigt. In einer Implementierung sammelt ein Gerät wie beispielsweise ein tragbares Band physiologische Informationen von seinem Träger. Die physiologischen Informationen können Herzfrequenz oder Atemfrequenz umfassen. Die physiologischen Informationen der Person werden durch einen Algorithmus klassifiziert, der durch maschinelle Lerntechniken abgeleitet wurde. Der Algorithmus wird trainiert, indem Daten von anderen Personen verwendet werden, die sowohl gesund als auch krank sind, und/oder mit früheren Lesungen der eigenen physiologischen Informationen der Person. Der Algorithmus wertet einen Wert der physiologischen Informationen der Person aus, um eine Wahrscheinlichkeit zu generieren, dass die Person gesund ist oder dass die Person in den nächsten Tagen wahrscheinlich krank wird und/oder Fieber entwickelt.

Die US 2017/035328 A1 beschreibt ein Verfahren, eine Vorrichtungen und ein System zum Erkennen eines Nutzers eines tragbaren Fitnessmonitors unter Verwendung von Informationen, die unter Verwendung des tragbaren Fitnessmonitors erfasst wurden. Dabei werden Bewegungsdaten, die von Bewegungssensoren des tragbaren Fitnessmonitors erfasst werden, dazu verwendet, den Nutzer zu erkennen oder zu authentifizieren.

Die der Erfindung zugrunde liegende Aufgabe wird jeweils mit den Merkmalen der unabhängigen Patentansprüche gelöst. Ausführungsformen der Erfindung sind in den abhängigen Patentansprüchen angegeben.

Die Erfindung stellt ein elektronisches System und ein Verfahren vor, welche eine Klassifikation des physiologischen Zustandes des Nutzers ermöglichen.

Handelsübliche Fitnesstracker zeigen während des Sports beispielsweise den Puls an, zählen die Schritte beim Laufen oder berechnen die geleisteten Kilokalorien. Im Gegensatz zu Ausführungsformen der vorliegenden Erfindung sind sie jedoch nicht dafür konzipiert bzw. konfiguriert, eine ausführliche Auswertung während des Trainings zu tätigen. Eine solche Auswertung umfasst zum Beispiel das Bestimmen eines Stresslevels, das Feststellen eines aufgewärmten Körpers für den Sport oder das frühzeitige Erkennen von Hinweisen auf eine mögliche Erkrankung oder Verletzung.

Die eingangs erwähnten Probleme hinsichtlich Fehler- und Störanfälligkeit können dadurch gelöst werden, dass eine Mehrzahl von Parametern erfasst und für die Klassifikation des physiologischen Zustandes verwendet wird. Somit können Einzelfehler besser kompensiert werden.

Das elektronische System umfasst ein mobiles, tragbares Kommunikationssystem, welches mindestens einen Sensor zur Erfassung von Daten, ein Klassifikationsmodul, einen Sensor und einen internen Speicher umfasst. Die Daten umfassen biometrischen Daten des Nutzers. Der Sensor kann beispielsweise einen Drucksensor, einen Temperatursensor, einen Schrittzähler oder einen anderen geeigneten Sensor zur Erfassung von biometrischen Daten umfassen.

Der Sensor des Kommunikationssystems umfasst einen internen Sensor zur Erfassung einer Bewegung des Kommunikationssystems, wobei die Bewegung des Kommunikationssystems durch eine grobmotorische Bewegung des aktuellen Nutzers des Kommunikationssystems verursacht wird, während der Nutzer das Kommunikationsgerät bei sich trägt. Die physiologischen Parameter können Parameter der Bewegung des Kommunikationssystems umfassen. Bei einer grobmotorischen Bewegung handelt es sich beispielweise um eine Bewegung, welche eine Bewegung eines oder mehrerer Gliedmaßen, d.h. eines oder zweier Beine und/oder eines oder zweier Arme, umfasst. Bei einer grobmotorischen Bewegung kann es sich ferner beispielweise um eine Bewegung handeln, welche eine Bewegung eines oder mehrerer Glieder eines oder mehrerer Gliedmaßen, z.B. einer oder zweier Hände und/oder eines oder zweier Unterarme, umfasst. Bei einer feinmotorischen Bewegung handelt es sich beispielweise um eine Bewegung eines oder mehrerer Finger. Ferner kann es sich bei einer feinmotorischen Bewegung beispielweise um eine Bewegung des Mundes, eines Augen und/oder eines oder mehrerer Gesichtsmuskeln handeln.

Ein mobiles, tragbares Kommunikationssystem kann ein einzelnes eigenständiges Gerät oder mehrere mechanische und/oder kommunikativ miteinander verbundene Geräte umfassen. Ein solches mobiles, tragbares Kommunikationssystem kann zum Beispiel umfassen: ein Smartphone, ein Tablet, ein Personal Digital Assistant, einen Pager, Smartglasses, eine Smartwatch, ein Navigationsgerät, einen Activity Tracker bzw. Fitnesstracker, ein Gerät zur Erfassung medizinischer Daten, insbesondere physiologischer und/oder biometrischer Daten wie ein Pulsmessgerät oder ein Blutdruckmessgerät. Die Klassifikation des physiologischen Zustandes lässt sich von allen mobilen und tragbaren Geräten und Systemen ausführen, die in der Lage sind, elektronische Daten zu verarbeiten und mindestens einen Sensor zur Erfassung von biometrischen Daten aufweisen.

Die biometrischen Daten umfassen dabei Messwerte für eine Mehrzahl von biometrischen Parametern.

Als biometrische Parameter werden Parameter bezeichnet, welche den Körper eines Nutzers und seine Funktionen quantifizieren. Dabei kann es sich beispielsweise um Eigenschaften des Körpers handeln, wie beispielsweise den Augenabstand, die Ohrmuschelform, die Schuhgröße oder dem Fingerabdruckmuster. Ferner können biometrische Parameter auch Parameter umfassen, die sich mit der Zeit ändern können. Das Körpergewicht beispielsweise kann sich im Laufe des Lebens eines Menschen ändern. Ferner können sich auch Änderungen von biometrische Parameter, z.B. Augenabstand, Schuhgröße etc., bei Jugendlichen im Zuge des Heranwachsens ergeben.

Ferner können die biometrischen Parameter Parameter einer Bewegung, etwa einer grobmotorischen Bewegung umfassen. Ein solcher Parameter kann einen konkreten Parameter, wie beispielsweise die Amplitude der Auf- und Abbewegung des Körpers beim Gehen, oder einen abstrakten Parameter, wie beispielsweise den durchschnittlichen Fehler der Hüftdrehung um die Körperlängsachse beim Laufen, umfassen.

Die biometrischen Daten können beispielsweise eine Pulsfrequenz, einen Blutdruck, eine Körpertemperatur, eine Schrittfrequenz oder andere biometrische Parameter umfassen. Das Klassifikationsmodul ist zur Erkennung einer Mehrzahl von generischen Zuständen einer Mehrzahl von physiologischen Parametern mithilfe von Trainingsdatensätzen einer Nutzerkohorte trainiert und wird durch den Prozessor des Kommunikationssystems ausgeführt.

Die physiologischen Parameter umfassen Parameter, die für eine Klassifikation des physiologischen Zustandes verwendet werden. Die physiologischen Parameter können die biometrischen Parameter eines Nutzers direkt oder eine Kombination aus mehreren biometrischen Parametern oder aus mindestens einem biometrischen und einem nichtbiometrischen Parametern umfassen. Ein nichtbiometrischer Parameter umfasst beispielsweise die Zeit bzw. eine Zeiteinheit. So können beispielsweise die Änderung des Pulses, die Laufgeschwindigkeit und/oder die Änderung der Laufgeschwindigkeit einen physiologischen Parameter bilden.

Ein physiologischer Zustand bezeichnet den Zustand des Körpers eines Nutzers. Der Nutzer kann ein Mensch oder ein anderes Lebewesen sein. Ein Zustand wird dabei durch N physiologische Parametern charakterisiert, wobei N größer 1 ist. Die verschiedenen Zustände bilden Parameterwolken in einem N-dimensionalen Parameterraum. Diese Wolken können klar unterscheidbar voneinander sein oder einander partiell oder ganz überlappen. Versucht man den physiologischen Zustand eines Nutzers zu charakterisieren, so umfasst die Klassifikation eine Zuordnung der zur Verfügung stehenden physiologischen Parameter zu einem Zustand bzw. zu einer Zustandswolke.

Das Klassifikationsergebnis umfasst dabei einen oder mehrere Zustände, welche sogenannte generische Zustände sind. Die generischen Zustände sind bekannt und hinreichend beschrieben, um sie anhand der physiologischen Parameter klassifizieren zu können. Ferner wurden die physiologischen Parameter für jeden dieser Zustände als Datensätze einer Nutzerkohorte, sogenannte Trainingsdatensätze, erfasst, wodurch die generischen Zustände in Kombination mit den Trainingsdatensätzen eine Grundlage für die Klassifikation des physiologischen Zustandes bilden.

Unter einem Referenzzustand wird hier ein physiologischer Zustand verstanden, welcher als Bezugspunkt und/oder Bezugsbereich für den Vergleich mit allen klassifizierbaren physiologischen Zuständen verwendet wird. Der Referenzzustand kann beispielsweise dadurch definiert sein, dass eine Mehrzahl von Parametern in einem Normalbereich ist. Der Normalbereich der Parameter ist beispielsweise definiert als ein Bereich in welchem die Parameter einer Mehrheit von Nutzern einer repräsentativen Gruppe von Durchschnittsnutzern liegen. Der Normalbereich kann ferner beispielsweise auf den Nutzer individuell angepasst und/oder unabhängig durch Fachpersonal, beispielsweise einen Arzt, einen Fitnesstrainer etc., festgelegt sein. Unter einem Anormalzustand wird hier ein physiologischer Zustand verstanden, welcher von dem Referenzzustand abweicht, d.h. bei welchem einer oder mehrere der Parameter der Mehrzahl von Parametern außerhalb des Normalbereichs liegt. Der klassifizierte physiologische Zustand kann dabei einer Gruppe von Zuständen zugeordnet werden, die entweder akzeptable Zustände umfasst oder der klassifizierte physiologische Zustand kann einer Gruppe von Zuständen zugeordnet werden, die inakzeptable Zustände umfasst. Inakzeptable Zustände können beispielsweise für den Nutzer problematische und/oder schädliche Zustände umfassen. Akzeptable Zustände können beispielsweise für den Nutzer unproblematische und/oder vorteilhafte Zustände umfassen.

Beispielsweise ein Mensch, welcher ein Fitnesstraining absolviert, macht sich zunächst warm. Der Körper befindet sich zunächst in seinem Referenzzustand. Durch die Bewegung steigt der Puls, die Muskulatur wird weicher. Der Körper wechselt in einen "Trainingszustand". Der Trainingszustand kann beispielsweise ein akzeptabler Zustand sein. Je nach Trainingsart wechselt der Körper mit der Zeit in einen Zustand, in dem Fett verbrannt, Kondition aufgebaut und/oder Muskulatur aufgebaut wird. Ein weiteres Beispiel für mögliche Zustände sind Kreislaufstörungen. Bewegt sich der Nutzer sehr plötzlich mit sehr hoher Intensität, beispielsweise weil der Nutzer schnell zu einer Bushaltestelle läuft, um einen gerade anhaltenden Bus nehmen zu können, so kann sich der Körper in einer so kurzen Zeit möglicherweise nicht umstellen und dem Körper kann im Extremfall ein Kreislaufzusammenbruch, beispielsweise aufgrund von Unterzuckerung, drohen. Eine solche Kreislaufstörung kann beispielsweise als ein inakzeptabler Zustand klassifiziert werden. Auch äußere Einflüsse können Auswirkungen auf den physiologischen Zustand des Nutzers haben. Insbesondere an heißen Tagen bzw. in heißer Umgebung kann beispielsweise ein Sonnenstich einen von dem Referenzzustand abweichenden Zustand verursachen. Die Klassifikation eines solchen Zustands kann beispielsweise ein inakzeptabler Zustand sein.

Zur Klassifikation des physiologischen Zustandes führt das System die folgenden Schritte aus:
- Erfassen der Daten durch den mindestens einen Sensor.
- Eingeben der Daten in das Klassifikationsmodul.
- Generieren eines Klassifikationsergebnisses aus den Daten durch das Klassifikationsmodul.
- Speichern des Klassifikationsergebnisses in dem Speicher des Kommunikationssystems.

Das Klassifikationsergebnis umfasst ein aktuelles Gesundheitsprofil des Nutzers. Das Gesundheitsprofil umfasst eine Klassifikation des physiologischen Zustandes des Nutzers hinsichtlich der Mehrzahl von generischen Zuständen der Mehrzahl physiologischer Parameter.

Die Klassifikation des aktuellen physiologischen Zustandes des Nutzers umfasst ein Echtzeitabbild des aktuellen physiologischen Zustandes und mindestens eine Zuordnung des physiologischen Zustandes in eine Klasse von Zuständen und/oder zu einem einzelnen Zustand.

In einer Ausführungsform umfasst mindestens einer der generischen Zustände der Mehrzahl von physiologischen Parametern einen Anormalzustand des Nutzers. Als Anormalzustand wird hierin eine Abweichung des physiologischen Zustandes von dem Referenzzustand betrachtet, welcher eine Schädigung des Körpers des Nutzers, seines Immunsystems, eine Verletzung, eine Erkrankung aufgrund eines Virus, eines Bakteriums und/oder eines Parasiten und/oder einen beliebigen negativen physiologischen Zustand des Nutzers andeutet. Wird gemäß dem Klassifikationsergebnis ein Anormalzustand klassifiziert, so ist dies ein Hinweis darauf, dass der Körper des Nutzers eine Auffälligkeit zeigt. Der Nutzer kann beispielsweise diese Klassifikation als Warnung betrachten und daraufhin einen Arzt aufsuchen, um sich bezüglich des klassifizierten physiologischen Zustandes untersuchen zu lassen. Das Klassifikationsergebnis kann dem Arzt somit Auffälligkeiten aufzeigen, wodurch dieser eine Diagnose eventuell schneller zu stellen vermag.

Bewegt sich beispielsweise ein Nutzer und ein grobmotorisches Bewegungsmusterregistriert dabei kontinuierlich seine Bewegung, so erhöht sich der Puls des Nutzers. Das Klassifikationsmodul erkennt einen erhöhten Puls bei gleichzeitiger Bewegung und klassifiziert den physiologischen Zustand als einen Bewegungszustand. Ist hingegen der Puls erhöht, ohne dass sich der Nutzer bewegt bzw. eine grobmotorische Bewegung erkannt wird, so könnte beispielsweise eine Entzündung im Körper des Nutzers vorliegen Dem Nutzer würde diese Auffälligkeit mittels seines Gesundheitsprofils mitgeteilt werden.

In einer Ausführungsform verwendet das Klassifikationsmodul ältere Daten zur Generierung des Klassifikationsergebnisses. Älter bezeichnet in diesem Zusammenhang, dass die Daten schon für eine vorangegangene Klassifikation des physiologischen Zustandes verwendet wurden.

Durch das Verwenden älterer Daten können einzelne physiologische Parameter in einem zeitlichen Kontext betrachtet werden. Ist der Nutzer beispielsweise gerade am Ende einer Trainingseinheit angelangt, so kann gemäß dieser Ausführungsform eine erhöhte Pulsfrequenz in einem Kontext der Trainingseinheit betrachtet werden. Üblicherweise benötigt der Körper eine gewisse Zeit, um sich wieder in den Referenzzustand zu begeben. Die Betrachtung des zeitlichen Kontexts berücksichtigt dies. Würde der Puls beispielsweise ohne zeitlichen Kontext betrachtet werden, so würde das Klassifikationsergebnis in den ersten Momenten nach einer Trainingseinheit einen viel zu hohen Ruhepuls erfassen, da sich der Nutzer nicht mehr bewegt.

In einer Ausführungsform umfassen die biometrischen Daten ferner Daten einer grobmotorischen Bewegung des Nutzers, wobei die Mehrzahl von physiologischen Parametern Parameter einer grobmotorischen Bewegung umfassen.

Viele mobile, tragbare Kommunikationssysteme, wie Smartphones, sind heute ohnehin mit Sensoren ausgestattet, welche eine Lage des Geräts im Raum erfassen können, wodurch sich zum Beispiel die Anzeige auf dem Bildschirm in die richtige Position relativ zur räumlichen Orientierung des Geräts drehen kann. Ein solcher Sensor ist üblicherweise ein Beschleunigungssensor, ein Gyroskop oder eine Kombination aus beiden. Es ist nicht nur möglich, mit diesem Sensor die Raumlage des mobilen, tragbaren Kommunikationssystems, sondern auch eine grobmotorische Bewegung des Nutzers zu erfassen, wodurch der Sensor als Bewegungssensor verwendet werden kann.

Eine grobmotorische Bewegung bezeichnet dabei alle Bewegungsfertigkeiten, die ein Mensch mit seinen Gliedmaßen, seinem Rumpf und seinem Kopf erlernen kann. Dabei werden Hauptmuskelgruppen beansprucht. Fertigkeiten der Grobmotorik sind beispielhaft Gehen, Joggen, Rennen, Hüpfen, Fahrradfahren oder Autofahren. Auch das Bewegen des Armes, um eine Verrichtung, wie beispielsweise ein Heben eines Glases zum Trinken oder Essen, auszuführen, kann ebenso wie eine Armbewegung, um ein Mobiltelefon aus der Tasche zu ziehen, als grobmotorische Bewegung aufgefasst werden. Demgegenüber gilt ein Greifen einer Tasse als feinmotorische Bewegung, da die Bewegung zum Greifen mit den Fingern ausgeführt wird und feinere Muskelgruppen beansprucht. Dabei kann eine grobmotorische Bewegung insbesondere auch eine Hüpfbewegung des Nutzers umfassen.

Durch das Verwenden einer grobmotorischen Bewegung zur Klassifikation eines physiologischen Zustandes lassen sich auch Zustände klassifizieren, die Einfluss auf die grobmotorische Bewegung des Nutzers haben. Das Verwenden von Parametern einer grobmotorischen Bewegung zur Klassifikation des physiologischen Zustandes erhöht ferner die Wahrscheinlichkeit einer korrekten Klassifikation des physiologischen Zustandes. Da sich jeder Mensch auf seine eigene Art bewegt und die Parameter, welche eine grobmotorische Bewegung beschreiben, variieren können sobald sich der physiologische Zustand des Nutzers ändert, kann eine Änderung dieser Parameter auf eine Abweichung von dem Referenzzustand des Nutzers hindeuten. Beispielsweise kann ein sich anbahnender Grippeinfekt das Bewegungsmuster des Nutzers beim Joggen beeinflussen. Der Körper reagiert durch die anwachsende Aktivität des Immunsystems mit weniger Leistung bei der Bewegung. Das Abfallen der Leistung hat beispielsweise kürzere Schritte oder ein geringeres Abheben des Fußes vom Boden zur Folge. Kürzere Schritte und geringere Abhebehöhen der Füße resultieren wiederum beispielsweise in einer Änderung der Hüftbewegung, welche von einem Kommunikationssystem in der Tasche des Nutzers erfasst wird.

Je nachdem welcher Parameter einer grobmotorischen Bewegung sich ändert und wie sich dieser Parameter ändert sind aus der Änderung gegenüber dem Referenzzustand entsprechende Klassifikationen möglich. Werden beispielsweise die Schritte mit einem Fuß kürzer als mit dem anderen, so umfasst beispielsweise das Klassifikationsergebnis eine Verletzung in dem entsprechenden Bein. Ist gleichzeitig die Pulsfrequenz gegenüber der üblichen Pulsfrequenz beim Laufen erhöht, so kann beispielsweise die Kombination mit der Beinverletzung darauf hindeuten, dass eines der Gelenke des betreffenden Beines eine Entzündung aufweist.

In einer Ausführungsform umfassen die biometrischen Daten ferner Daten einer feinmotorischen Bewegung, wobei die physiologischen Parameter Parameter einer feinmotorischen Bewegung umfassen.

Eine feinmotorische Bewegung ist eine Bewegung feiner Muskelgruppen, wie beispielsweise die Muskulatur der Finger. Feinmotorik bezeichnet gezielte und koordinierte Bewegungen, beispielsweise von Hand- und/oder Fingermuskulatur, aber auch den Muskeln des Mundes, der Augen und des Gesichtes. Die feinmotorische Bewegung, welche durch einen feinmotorischen Sensor des Kommunikationssystems erfasst wird, kann etwa eine bestimmte Bewegung der Finger umfassen.

Nach Ausführungsformen der Erfindung werden durch den Sensor zur Erfassung einer feinmotorischen Bewegung die Eingabegeschwindigkeit, der Eingabetakt und/oder die Eingabegenauigkeit des Nutzers erfasst, während er die Eingabe in das Kommunikationssystem tätigt. Eine solche Eingabe kann beispielsweise, ohne darauf beschränkt zu sein, das Tippen von Wörtern bzw. Wischen von Wörtern, das heißt einem zum Tippen analogen Eingabeverfahren, bei welchem der oder die Finger bei Auswahl von Buchstaben in Kontakt zur Bildschirmschirmoberfläche jedoch beibehalten wird, auf einer virtuellen Tastatur, das Nachfahren von auf dem Bildschirm gezeigten geometrischen Figuren oder einer anderen Bewegung, mit der der Nutzer eine Eingabe tätigt, umfassen. Ferner können feinmotorische Bewegungen Änderungen der Ausrichtung, wie etwa des Neigungswinkels des Kommunikationssystems während der Nutzung umfassen. Ein Sensor zur Erfassung einer feinmotorischen Bewegung kann beispielsweise als optisches System oder als ein Touchpad bzw. ein Touchscreen, insbesondere, jedoch ohne darauf beschränkt zu sein, ein resistiver Touchscreen, ein oberflächenkapazitiver Touchscreen, ein projiziert kapazitiver Touchscreen oder ein induktiver Touchscreen ausgebildet sein.

Durch das Verwenden einer feinmotorischen Bewegung zur Klassifikation eines physiologischen Zustandes lassen sich Zustände klassifizieren, die Einfluss auf die feinmotorische Bewegung des Nutzers haben. Beispielsweise der physiologische Zustand von an Parkinson und/oder Epilepsie erkrankten Nutzern lässt sich durch die Erfassung von feinmotorischen Bewegungen klassifizieren. Ein Zittern einzelner Gliedmaßen oder eine Genauigkeit der Eingabe in das Kommunikationssystem können beispielsweise Indikatoren für einen Zustand eines an Parkinson oder an Epilepsie erkrankten Nutzers umfassen.

In einer Ausführungsform umfasst das Generieren des Klassifikationsergebnisses ein Berechnen einer Mehrzahl nutzerspezifischer Parameter unter Verwendung der biometrischen Daten, wobei die Mehrzahl spezifischer Parameter mit der Mehrzahl physiologischer Parameter der generischen Zustände verglichen wird, wobei der Vergleich der Parameter miteinander den physiologischen Zustand des Nutzers hinsichtlich der Mehrzahl von generischen Zuständen von physiologischen Parametern klassifiziert.

Nach Ausführungsformen umfasst das Kommunikationssystem Anwendungen, wobei die Daten ferner Anwendungsdaten des Nutzers umfassen, wobei aus den Anwendungsdaten Anpassungsparameter bestimmt werden, welche externe Einflussfaktoren der biometrischen Daten beschreiben, wobei die Anpassungsparameter bei der Generierung des Klassifikationsergebnisses zur Anpassung der Klassifikation an die Einflussfaktoren verwendet werden.

Durch das Verwenden der Anwendungsdaten und das damit verbundene Verwenden der Anpassungsparameter zur Generierung des Klassifikationsergebnisses ergibt sich in vorteilhafter Weise, dass das Klassifikationsergebnis an externe Einflussfaktoren angepasst werden kann. Befindet sich der Nutzer beispielsweise in einer Umgebung mit einer hohen oder niedrigeren Lufttemperatur und wird die Körpertemperatur mittels eines Sensors an einem Handgelenk gemessen, so kann die Messung durch die Lufttemperatur beeinflusst werden. Die gemessene Temperatur an dem Handgelenk kann beispielsweise unter einem Durchschnittswert liegen, falls sich der Nutzer in einer kalten Umgebung befindet. Durch das Verwenden von beispielsweise Wetterdaten, welche die aktuelle Lufttemperatur umfassen, kann die gemessene Temperatur unter Korrektur der Wetterdaten mit der Temperatur des Referenzzustandes verglichen werden. In einem weiteren Beispiel ändert sich der Blutdruck des Nutzers, wenn er sich in einer anderen Höhe über dem Meeresspiegel als gewöhnlich aufhält. Dies kann beispielsweise durch ein Barometer erfasst oder durch einen GPS- oder Galileo-Signal bestimmt werden und somit die Auswertung des Blutdrucks des Nutzers korrigieren.

Je nachdem welche Anwendungen ausgeführt werden, lassen sich eine Mehrzahl von physiologischen Parametern korrigieren. Insbesondere kann die Kombination von verschiedenen Einflussfaktoren einzelne und/oder eine Mehrzahl von physiologischen Parametern korrigieren. Beispielsweise können Luftfeuchtigkeit und Luftdichte, wobei letztere durch die aktuelle Höhe über dem Meeresspiegel errechnet wird, das Verhältnis von Puls und Atemfrequenz zur Bewegungsgeschwindigkeit beeinflussen. Ist der Einfluss bekannt, kann eine entsprechende Korrektur vorgenommen werden.

In einer Ausführungsform umfassen die Anwendungsdaten:
- Positionsdaten des Kommunikationssystems, welche durch ein Verfahren zur Positionsbestimmung durch einen Sensor zur Bestimmung der Position des Kommunikationssystems erfasst werden, und/oder
- Anwendungsnutzungsdaten des Nutzers, und/oder
- Verbindungsdaten des Kommunikationssystems mit anderen Geräten und/oder
- Kalender- und/oder Uhrzeitdaten in einer in dem Kommunikationssystem implementierten Uhr oder einer externen Uhr, deren Signal durch einen Sensor des Kommunikationssystems empfangen wird und/oder
- Umgebungsdaten, welche mittels mindestens einem internen Umgebungssensor oder mittels mindestens einem externen Umgebungssensor erfasst werden, dessen Signal durch einen Sensor des Kommunikationssystems empfangen wird.

Die Positionsdaten des Kommunikationssystems werden durch ein Verfahren zur Positionsbestimmung durch einen Positionssensor des Kommunikationssystems erfasst. Ein solches Verfahren kann zum Beispiel ein Erfassen eines GPS-Signals, eines Galileo-Signals oder einer triangulierten Position aus WLAN-Verbindungsdaten oder Verbindungsdaten eines sonstigen Funknetzes umfassen, welches Funkzellen aufweist, wie etwa ein Mobilfunknetz.

Die Positionsdaten können beispielsweise dazu verwendet werden, den Luftdruck bzw. die lokalen Wetterdaten bei der Klassifikation des physiologischen Zustandes zu berücksichtigen. Beispielsweise können sich die Außentemperatur und/oder die Höhe der Position des Nutzers über dem Meeresspiegel auf den physiologischen Zustand des Nutzers auswirken.

Die Positionsdaten können beispielsweise dazu verwendet werden die Umgebungsdaten, wie beispielsweise den Luftdruck bzw. die lokalen Wetterdaten bei der Klassifikation des physiologischen Zustandes zu berücksichtigen. Beispielsweise können sich die Außentemperatur und/oder die Höhe der Position des Nutzers über dem Meeresspiegel auf den physiologischen Zustand des Nutzers auswirken. Ferner kann durch die Kombination von Positionsdaten mit der Uhrzeit beispielsweise eine Bewegungsgeschwindigkeit ermittelt werden oder durch die Kombination von Positionsdaten mit Kalenderdaten beispielsweise ein Wechsel der umgebenden Klimazone festgestellt werden, welcher sich ebenfalls auf den Körper auswirken kann.

Kalender- und/oder Uhrzeitdaten können durch eine in dem Kommunikationssystem implementierte Uhr oder eine externe Uhr, deren Signal durch einen Sensor, insbesondere ein Funksignal durch einen Funksensor, des Kommunikationssystems empfangen wird, erfasst werden. In einer weiteren Ausführungsform der Erfindung werden die biometrischen Daten mit den Kalender- und/oder Uhrzeitdaten oder anderen nicht-biometrischen Daten, wie beispielsweise den Verbindungsdaten, dem Umgebungsdaten und/oder den Positionsdaten korreliert. Bei einer Korrelation der biometrischen Daten mit den Kalenderdaten kann beispielsweise eine Ursache für einen erhöhten Puls und/oder Blutdruck in Form eine Stresssituation identifiziert werden.

Umgebungsdaten, welche mittels mindestens einem internen Umgebungssensor oder mittels mindestens einem externen Umgebungssensor erfasst werden, dessen Signal durch einen Sensor des Kommunikationssystems empfangen wird, können beispielsweise die Umgebungstemperatur, die Windstärke, ein umgebendes Strahlungsniveau und/oder die lokale Beschleunigung umfassen.

In einer Ausführungsform signalisiert das Kommunikationssystem dem Nutzer mittels eines Signals, dass die Klassifikation des physiologischen Zustandes einen physiologischen Zustand außerhalb des Normalbereichs umfasst.

Das Signalisieren einer Klassifikation des physiologischen Zustandes außerhalb des Referenzzustandes bewirkt in vorteilhafter Weise, dass sich der Nutzer gemäß der Abweichung des physiologischen Zustandes von dem Referenzzustand verhalten kann. So kann beispielsweise ein in einem Trainingsprozess befindlicher Nutzer auf das Signal hin eine Aufwärmübung beenden und zu einer eigentlichen Trainingsübung wechseln. Ein Nutzer, welcher die Erfindung nutzt, um seine Gesundheit bezüglich einer Krankheit zu überwachen, beispielsweise weil er gefährdet bzw. ein Risikopatient ist, kann durch ein Signal gewarnt sein, dass sein physiologischer Zustand von dem Referenzzustand abweicht. Auf das Signal hin kann der Nutzer beispielsweise einen Arzt oder Apotheker kontaktieren und/oder Medikamente einnehmen.

In einer Ausführungsform umfasst das Signal das Ergebnis der Klassifikation des physiologischen Zustandes.

Das Signalisieren des Ergebnisses des physiologischen Zustandes bewirkt in vorteilhafter Weise, dass der Nutzer seinen physiologischen Zustand bezüglich einer Vielzahl von möglichen Klassifikationsergebnissen überwachen kann. Der trainierende Nutzer kann, nachdem er mit der Aufwärmübung und der eigentlichen Trainingsübung fertig ist, eine Dehnübung beginnen, um den Körper wieder in einen Referenzzustand zu bringen. Um den optimalen Trainingseffekt zu erzielen, ist es jedoch notwendig zu wissen, wann der Nutzer einen physiologischen Zustand erreicht hat, indem sein Training die höchste Effizienz aufweist. Da sich dieser Zustand von dem Zustand des aufgewärmten Körpers unterscheiden kann, muss der Nutzer wissen, welchen Zustand sein Körper gerade aufweist. Dies wird ihm durch das Signal vermittelt.

In einem weiteren Beispiel versucht ein Nutzer Sport zu machen, ohne zu bemerken, dass er an einer Krankheit, beispielsweise einer Erkältung, erkrankt ist. Mit der Signalisierung, dass der Trainingszustand nach dem Aufwärmen erreicht ist, wird dem Nutzer zusätzlich signalisiert, dass ein Risiko für eine Erkrankung, insbesondere eine Erkältung, besteht. Das Kommunikationssystem ist gemäß dieser Ausführungsform in der Lage zwei oder eine Mehrzahl von physiologischen Zuständen einzeln oder parallel zu signalisieren. Beispielsweise können mehrere Anormalzustände parallel signalisiert werden.

Das Signal kann ein akustisches, optisches oder haptisches Signal umfassen. Beispielsweise kann das Signal mittels eines Lautsprechers einen Ton oder eine Tonfolge wiedergeben, welche einem physiologischen Zustand zugeordnet ist bzw. welche einer Mehrzahl von physiologischen Zuständen zugeordnet sind. In einer weiteren Ausführungsform wird dem Nutzer über Sprache das Klassifikationsergebnis signalisiert. Die Sprache kann dabei zuvor eingesprochen oder computergeneriert sein. Das Signal kann beispielsweise ferner über ein Anzeigemittel, insbesondere einen Bildschirm, eine Lampe, eine Leuchtdiode oder andere Leuchtmittel dargestellt werden. In Ausführungsformen sind jedem physiologischen Zustand Farben, Farbmuster und/oder Blinkmuster zugeordnet. Das Signal kann beispielsweise ferner ein oder eine Mehrzahl von Vibrationsmustern umfassen, welche einem oder einer Mehrzahl von physiologischen Zuständen zugeordnet sind.

In einer Ausführungsform ist das Kommunikationssystem dazu konfiguriert, einen Notruf abzusetzen, falls das Klassifikationsergebnis eine kritische Klassifikation des physiologischen Zustandes des Nutzers umfasst.

Eine kritische Klassifikation des physiologischen Zustandes umfasst dabei einen Zustand, in dem der Nutzer Hilfe benötigt. Ein solcher Zustand kann beispielsweise einen Zusammenbruch des Herz-Kreislauf-Systems, einen Herzinfarkt, einen Schlaganfall, einen epileptischen Anfall oder eine Situation umfassen, in welcher der Nutzer die Hilfe eines Arztes oder eines vergleichbaren Heilberufes benötigt.

Durch das Absetzen eines Notrufs, weil das Klassifikationsergebnis eine kritische Klassifikation des physiologischen Zustandes umfasst, kann frühestmöglich Hilfe gerufen werden. Insbesondere Herzversagen, Schlaganfälle, Herzinfarkte oder allergische Reaktionen können letal sein, falls nicht rechtzeitig Hilfe gerufen wird. Das automatische Absetzen eines Notrufs kann damit das Risiko von letalen Folgen eines derartigen physiologischen Zustandes vermindern.

Der Notruf kann in Ausführungsformen ein akustisches Signal umfassen. Umfasst das Klassifikationsergebnis eine kritische Klassifikation des physiologischen Zustandes, so beginnt beispielsweise eine Sirene oder ein Lautsprecher, einen Alarm auszusenden, um Umstehende und/oder in der Nähe befindliche Personen auf den Nutzer aufmerksam zu machen. In einer weiteren Ausführungsform wird der Notruf über ein Mobilfunknetz abgesetzt. Der Notruf kann beispielsweise direkt an eine Notrufzentrale gerichtet sein. In einer weiteren Ausführungsform umfasst der Notruf mindestens die Position des Nutzers, das Klassifikationsergebnis und/oder Informationen bezüglich der Identität des Nutzers.

In einer Ausführungsform weist das Kommunikationssystem eine Schnittstelle auf, wobei das Kommunikationssystem dazu konfiguriert ist, über die Schnittstelle mit einem elektronischen Heilberufsausweis (eHBA) zu kommunizieren. Ein Besitzer eines eHBA erlangt Zugriff auf die gespeicherten Gesundheitsprofile des Nutzers, wobei das Erlangen des Zugriffs die folgenden Schritte umfasst:
- Aufbauen einer kryptografisch gesicherten Verbindung zwischen dem eHBA und dem Kommunikationssystem.
- Authentifizieren des Besitzers des eHBA.
- Prüfen, ob ein kritischer physiologischer Zustand des Nutzers vorliegt.
- Gewähren des Zugriffs auf die gespeicherten Gesundheitsprofile des Nutzers durch das Kommunikationssystem, falls das Klassifikationsergebnis einen kritischen physiologischen Zustand des Nutzers umfasst.

Der eHBA umfasst beispielsweise einen Chip, welcher mittels Drahtloskommunikation mit dem Kommunikationssystem kommunizieren kann und/oder ein Zertifikat einer Berechtigungs-PKI (Public Key Infrastruktur), welches dem Besitzer des eHBA den Zugriff auf die Gesundheitsprofile ermöglicht. Dabei kann der Zugriff je nach Konfiguration des eHBA auf bestimmte Parameter beschränkt sein. Der Chip umfasst Daten in gespeicherter Form, welche den Besitzer des eHBA als qualifizierten Helfer identifizieren. Qualifizierte Helfer können beispielsweise Sanitäter, Ärzte, Polizisten, Feuerwehrleute und/oder ausgebildete Ersthelfer umfassen.

Das Zugreifen eines Besitzers eines eHBA auf die Gesundheitsprofile des Nutzers, falls die Klassifikation des physiologischen Zustandes einen kritischen physiologischen Zustand umfasst, bewirkt in vorteilhafter Weise, dass ein qualifizierter Helfer schnell Zugriff auf hilfreiche Informationen bezüglich des aktuellen physiologischen Zustandes des Nutzers erlangt. Insbesondere in kritischen Situationen kann das Vorhandensein von wichtigen Informationen darüber entscheiden, welche Behandlung des Nutzers anzuwenden ist. Die wichtigen Informationen können beispielsweise Informationen bezüglich Allergien, Unverträglichkeiten, bereits bekannten Vorerkrankungen und/oder Risiken für bestehende Krankheiten umfassen.

Das Verwenden eines eHBAs bzw. das Authentifizieren des Nutzers eines eHBAs bewirken in vorteilhafter Weise, dass der Zugriff auf die Gesundheitsprofile auf Personen eines Heilberufes beschränkt ist. Dadurch werden geltende Bestimmungen des Datenschutzes berücksichtigt und die Daten vor unerlaubten Zugriff geschützt.

In einer Ausführungsform weist das Kommunikationssystem ein Ausgabemittel auf, wobei das Ausgabemittel medizinische Informationen bezüglich des Nutzers, Informationen bezüglich des Klassifikationsergebnisses und/oder Informationen bezüglich anzuwendender Sofortmaßnahmen für eine Ersthilfe anzeigt, falls das Klassifikationsergebnis einen kritischen physiologischen Zustand des Nutzers umfasst.

Das Anzeigen von medizinischen Informationen bezüglich des Nutzers, Informationen bezüglich des Klassifikationsergebnisses und/oder Informationen bezüglich Sofortmaßnahmen bewirkt in vorteilhafter Weise, dass in Bezug auf Sofortmaßnahmen ungeschulte Personen dem Nutzer effizient helfen können. Die Informationen können beispielsweise eine Anleitung zur Ersthilfe, den Weg zu einem nahegelegenen Krankenhaus, einer Apotheke, einer Notfalleinrichtung, medizinischen Hilfsmitteln, wie etwa einem Defibrillator, oder wichtige Informationen, wie oben aufgelistet, umfassen.

Das Ausgabemittel kann beispielsweise einen Lautsprecher und/oder einen Bildschirm umfassen. Über das Ausgabemittel werden Instruktionen zur ersten Hilfe wiedergegeben. In einer Ausführungsform gibt ein Lautsprecher gesprochene Kommandos zur ersten Hilfe wieder, während ein Bildschirm die Kommandos als Piktogramm und/oder Text anzeigt.

Auf die Eingabe der Daten in das Klassifikationsmodul hin wird eine verhaltensbasierte Authentifizierung des Nutzers durchgeführt, wobei in dem Speicher des Kommunikationssystems mindestens eine Musterfunktion und ein Vergleichsdatensatz gespeichert sind, wobei die verhaltensbasierte Authentifizierung folgende Schritte umfasst:
- Vergleichen der Daten mit der mindestens einen Musterfunktion,
- Generieren von Klassifikationsparametern aus dem Vergleich der Daten mit der Musterfunktion,
- Generieren von Vergleichsparametern aus dem Vergleichsdatensatz,
- Vergleichen der Klassifikationsparameter mit den Vergleichsparametern,
- Generieren eines ersten Konfidenzwertes aus dem Vergleich der Klassifikationsparameter mit den Vergleichsparametern,
wobei der erste Konfidenzwert eine Wahrscheinlichkeit umfasst, mit der der Nutzer als der in dem Kommunikationssystem registrierte Nutzer identifiziert wird, wobei das Generieren des Klassifikationsergebnisses nur dann durchgeführt wird, falls der erste Konfidenzwert einen Schwellenwert überschreitet.

Das Verfahren zur verhaltensbasierten Authentifizierung eines Nutzers gegenüber einem mobilen, tragbaren Kommunikationssystem, welches ein Grobmotorikklassifikationsmodul aufweist, wobei das Grobmotorikklassifikationsmodul für eine Klassifikation grobmotorischer Messdaten konfiguriert und auf ein Erkennen einer grobmotorischen Bewegung eines registrierten Nutzers trainiert ist, umfasst:
- Wiederholtes Ausführen der Schritte:
   o Erfassen der grobmotorischen Messdaten durch mindestens einen Sensor des mobilen, tragbaren Kommunikationssystems,
   o Eingabe der grobmotorischen Messdaten in das Grobmotorikklassifikationsmodul,
   o Generieren eines Klassifikationsergebnisses durch das Grobmotorikklassifikationsmodul, ob der Nutzer ein in dem mobilen, tragbaren Kommunikationssystem registrierte Nutzer ist,
   o Speichern des Klassifikationsergebnisses in dem Speicher des mobilen, tragbaren Kommunikationssystems,
- auf eine Authentifizierungsanfrage hin, Zugreifen auf den Speicher des mobilen, tragbaren Kommunikationssystems, um mindestens eines der gespeicherten Klassifikationsergebnisse aus dem Speicher auszulesen,
- Auslesen und Auswerten des mindestens einen Klassifikationsergebnisses gemäß einem vorgegebenen Prüfungskriterium,
- Erzeugen eines Authentifizierungssignals, falls das Prüfungskriterium erfüllt ist, wobei das Authentifizierungssignal eine erfolgreiche Authentifizierung des aktuellen Nutzers signalisiert.

In einem Speicher des tragbaren Kommunikationssystems sind mindestens ein Muster in Form einer Musterfunktion und mindestens ein Vergleichsdatensatz gespeichert, wobei der Vergleichsdatensatz Werte für mindestens einen Vergleichsparameter beinhaltet, wobei das das Grobmotorikklassifikationsmodul auf die Eingabe der grobmotorischen Messdaten hin die folgenden Schritte ausführt:
- Vergleich der erfassten grobmotorischen Messdaten mit der mindestens einen Musterfunktion,
- Zuordnen der grobmotorischen Messdaten zu dem der Musterfunktion zugeordneten Muster und erhalten von mindestens einem dem ersten Muster entsprechenden Klassifikationsparameter, falls die grobmotorischen Messdaten dem mindestens einen, ersten Muster zugeordnet werden können,
- Errechnen eines Konfidenzwertes für jeden der Klassifikationsparameter durch einen Vergleich der Klassifikationsparameter mit dem jeweiligen Vergleichsparameter des Vergleichsdatensatzes und
- Generieren des auf den grobmotorischen Messdaten beruhenden Klassifikationsergebnisses aus den ersten Konfidenzwerten der Klassifikationsparameter.

Nach Ausführungsformen umfasst das Verfahren zur verhaltensbasierten Authentifizierung eines Nutzers gegenüber dem mobilen, tragbaren Kommunikationssystem, welches ferner ein Feinmotorikklassifikationsmodul aufweist, wobei das Feinmotorikklassifikationsmodul für eine Klassifikation feinmotorischer Messdaten konfiguriert und auf ein Erkennen einer feinmotorischen Bewegung eines registrierten Nutzers trainiert ist, beispielsweise:
- Wiederholtes Ausführen der Schritte:
   o Erfassen der feinmotorischen Messdaten durch mindestens einen Sensor des mobilen, tragbaren Kommunikationssystems,
   o Eingabe der feinmotorischen Messdaten in das Feinmotorikklassifikationsmodul,
   o Generieren eines weiteren Klassifikationsergebnisses durch das Feinmotorikklassifikationsmodul, ob der Nutzer ein in dem mobilen, tragbaren Kommunikationssystem registrierte Nutzer ist,
   o Speichern des weiteren Klassifikationsergebnisses in dem Speicher des mobilen, tragbaren Kommunikationssystems,
- beispielsweise auf eine Authentifizierungsanfrage hin, Zugreifen auf den Speicher des mobilen, tragbaren Kommunikationssystems, um mindestens eines der gespeicherten weiteren Klassifikationsergebnisse aus dem Speicher auszulesen,
wobei in die Auswertung des auf den grobmotorischen Messdaten beruhenden Klassifikationsergebnisses gemäß dem Prüfungskriterium auch das weitere auf den feinmotorischen Messdaten beruhenden Klassifikationsergebnis mit eingeht.

Nach Ausführungsformen sind in dem Speicher des mobilen, tragbaren Kommunikationssystems mindestens ein weiteres Muster in Form einer weiteren Musterfunktion und mindestens ein weiterer Vergleichsdatensatz gespeichert, wobei der weitere Vergleichsdatensatz Werte für mindestens einen weiteren Vergleichsparameter umfasst, wobei das Feinmotorikklassifikationsmodul auf die Eingabe der feinmotorischen Messdaten hin die folgenden Schritte ausführt:
- Vergleich der erfassten feinmotorischen Messdaten mit der mindestens einen weiteren Musterfunktion,
- Zuordnen der feinmotorischen Messdaten zu dem der weiteren Musterfunktion zugeordneten weiteren Muster und erhalten von mindestens einem dem weiteren Muster entsprechenden weiteren Klassifikationsparameter, falls die feinmotorischen Messdaten dem mindesten einen, weiteren Muster zugeordnet werden können,
- Errechnen eines Konfidenzwertes für jeden der weiteren Klassifikationsparameter durch einen Vergleich der weiteren Klassifikationsparameter mit dem jeweiligen weiteren Vergleichsparameter des weiteren Vergleichsdatensatzes,
- Generieren des auf den feinmotorischen Messdaten beruhenden Klassifikationsergebnisses aus den weiteren Konfidenzwerten der weiteren Klassifikationsparameter.

Nach Ausführungsformen ist das Prüfungskriterium erfüllt, falls:
- mindestens eines der Klassifikationsergebnisse, d.h. das auf den grobmotorischen Messdaten beruhenden Klassifikationsergebnis oder das auf den feinmotorischen Messdaten beruhenden Klassifikationsergebnis, einen durch das Prüfungskriterium vorgegebenen Schwellenwert überschreitet und/oder
- ein durch das Prüfungskriterium vorgegebenes Höchstalter der Klassifikationsergebnisse nicht überschritten wird und/oder
- eine Mindestanzahl an Klassifikationsergebnissen vorliegt, welche den Schwellenwert überschreitet.

Die verhaltensbasierte Authentifizierung des Nutzers vor oder parallel zu dem Generieren des Klassifikationsergebnisses bewirkt in vorteilhafter Weise, dass der physiologische Zustand des Nutzers nur dann erfasst wird, wenn er tatsächlich das Kommunikationssystem bei sich trägt und/oder verwendet. In Momenten, in denen der Nutzer das Kommunikationssystem nicht bei sich trägt bzw. in denen er das Kommunikationssystem nicht verwendet, werden ebenfalls Daten erfasst. Das Kommunikationssystem vergleicht die erfassten Daten mit dem Vergleichsdatensatz und bestimmt somit, ob der Nutzer das Kommunikationssystem nutzt und/oder bei sich trägt. Nutzt eine Person das Kommunikationssystem, welche nicht der registrierte Nutzer ist, beispielsweise weil der Nutzer das Kommunikationssystem der Person ausgehändigt hat oder weil die Person dem Nutzer das Kommunikationssystem entwendet hat, so unterscheiden sich die erfassten Daten ebenfalls von den Daten des Vergleichsdatensatzes.

Der Vergleichsdatensatz umfasst Daten des Nutzers, welche das Verhalten und/oder die individuelle Eigenschaft des Nutzers charakterisieren. Die Daten des Vergleichsdatensatzes und die erfassten Daten umfassen mindestens einen Parameter, welcher mindestens eine Eigenschaft des Nutzers und/oder seines Verhaltens quantifiziert. Der Vergleichsdatensatz umfasst die Daten des in dem Kommunikationssystem registrierten Nutzers. Die erfassten Daten umfassen die Daten des aktuellen Nutzers. Das Klassifikationsmodul generiert aus dem Vergleich der erfassten Daten mit dem Vergleichsdatensatz einen ersten Konfidenzwert, welcher den quantitativen Unterschied zwischen Klassifikationsparametern, generiert aus den erfassten Daten, und Vergleichsparametern, generiert aus dem Vergleichsdatensatz, ausdrückt. Der quantitative Unterschied lässt sich in eine Wahrscheinlichkeit übersetzen, mit der der Nutzer als der registrierte Nutzer identifiziert wird. Ist die bestimmte Wahrscheinlichkeit größer als ein vorgegebener Schwellenwert, so geniert das Klassifikationsmodul das Klassifikationsergebnis, da sich der Nutzer mittels der erfassten Daten authentifiziert hat. Über ein Festlegen des Schwellenwertes kann ein Kriterium für die Akzeptanz des Nutzers als registrierter Nutzer festgelegt werden.

Besonders vorteilhaft ist diese Ausführungsform, da die erfassten Daten eine doppelte Verwendung finden. Sie werden einerseits dazu verwendet den Nutzer gegenüber seinem Kommunikationssystem zu authentifizieren und andererseits dazu, den physiologischen Zustand des Nutzers zu klassifizieren.

Nach Ausführungsformen umfasst die Mehrzahl von generischen Zustände der Mehrzahl von physiologischen Parametern eine Auswahl von für die Authentifizierung zulässiger Zustände, wobei eine erfolgreiche Authentifizierung des Nutzers voraussetzt, dass der physiologische Zustand des Nutzers als ein zulässiger Zustand klassifiziert wird. Handelt es sich bei dem physiologischen Zustand um einen unzulässigen Zustand, beispielsweise weil der Nutzer ermüdet, körperlich geschwächt und/oder krank ist, wird eine Authentifizierung verweigert. Beispielsweise ist eine erfolgreiche Authentifizierung Voraussetzung für einen Zugriff und/oder Zutritt des Nutzers auf und/oder zu einem Fahrzeug, wie etwa ein Land-, Luft-, oder Wasserfahrzeug. Somit kann beispielsweise verhindert werden, dass ein übermüdeter, körperlich geschwächter und/oder kranker Nutzer ein entsprechendes Fahrzeug führt. Mithin kann das Risiko von Unfällen gesenkt werden. Beispielsweise muss es sich für eine erfolgreiche Authentifizierung bei dem Nutzer um einen registrierten Nutzer handeln, d.h. der erste Konfidenzwert muss einen entsprechenden Schwellenwert überschreiten, und der physiologische Zustand des Nutzers als ein zulässiger Zustand klassifiziert sein. Ist eines dieser beiden Kriterien nicht erfüllt, wird der Nutzer nicht erfolgreich authentifiziert. Beispielsweise wurde der Nutzer als ein registrierter Nutzer identifiziert, befindet sich jedoch in einem unzulässigen physiologischen Zustand, beispielsweise weil er ermüdet, körperlich geschwächt und/oder krank ist.

In einer Ausführungsform umfasst die verhaltensbasierte Authentifizierung ein Trainieren des Klassifikationsmoduls, sofern die verhaltensbasierte Authentifizierung erfolgreich war, wobei das Trainieren des Klassifikationsmoduls ein Hinzufügen der Daten zu dem Vergleichsdatensatz und ein Entfernen von Daten, welche älter als ein Schwellenwert sind, umfasst.

Das Trainieren des Klassifikationsmoduls bewirkt vorteilhafterweise, dass die verhaltensbasierte Authentifizierung auch dann angewendet werden kann, wenn sich das Verhalten des Nutzers ändert. Beispielsweise erleidet der Nutzer eine Verletzung im Bein oder in der Hand. Die Verletzung beeinflusst das Gehen bzw. das Schreiben und/oder Tippen des Nutzers, wodurch sich die diesen Vorgängen zugeordneten Parameter bzw. die entsprechenden Daten verändern. Ferner kann sich die Gangart oder das Tippverhalten des Nutzers über einen gewissen Zeitraum ohnehin auch ohne Verletzung ändern. Bei dem Vergleich der Daten mit dem Vergleichsdatensatz würden diese Änderungen mit der Zeit dazu führen, dass der Konfidenzwert sinkt, da der quantitative Unterschied zwischen den Daten und dem Vergleichsdatensatz zunimmt. Das Anpassen des Vergleichsdatensatzes an die aktuell erfassten Daten verhindert dies.

Das Trainieren des Klassifikationsmoduls kann sich in Ausführungsformen hinsichtlich der Klassifikation des physiologischen Zustandes des Nutzers von der Klassifikation hinsichtlich der Authentifizierung des Nutzers unterscheiden. Beispielsweise kann eine Beinverletzung die Authentifizierung beeinflussen, wobei sich durch das anpassen des Vergleichsdatensatzes der Nutzer trotz seiner Beinverletzung authentifizieren kann. Die Klassifikation des physiologischen Zustandes sollte sich demgegenüber nicht an den neuen Zustand anpassen. So lange der Nutzer eine Beinverletzung hat, wird ihm diese auch als Abweichung vom Referenzzustand angezeigt. Das Klassifikationsmodul wird in Bezug auf den physiologischen Zustand nicht mit dieser Abweichung auf eine Änderung des Referenzzustandes trainiert.

Würde hingegen beispielsweise der Nutzer an einem Langzeitkonditionstraining teilnehmen und mit der Zeit seine Kondition verbessern, so würde beispielsweise der Ruhepuls sinken. Diese Änderung würde ein Training des Klassifikationsmoduls bezüglich der Klassifikation des physiologischen Zustandes zur Folge haben. In Ausführungsformen wird das Klassifikationsmodul nur dann bezüglich des physiologischen Zustandes trainiert, wenn bestimmte physiologische Zustände klassifiziert wurden. Welche physiologischen Zustände dies umfasst, kann beispielsweise durch einen Arzt, einen Fitnesstrainer und/oder den Nutzer festgelegt werden.

Um den Vergleichsdatensatz an Veränderungen der Eigenschaften des Nutzers bzw. seines Verhaltens anzupassen, werden die aktuell erfassten Daten zu dem Vergleichsdatensatz hinzugefügt, und Daten, welche ein bestimmtes Schwellenalter erreicht haben, aus dem Vergleichsdatensatz entfernt. Durch das Entfernen der Daten aus dem Vergleichsdatensatz wird sichergestellt, dass alle Daten, welche das Verhalten und/oder Eigenschaften des Nutzers nicht mehr repräsentieren, nicht zur verhaltensbasierten Authentifizierung beitragen. Das Schwellenalter kann beispielsweise eine Woche, einen Monat, drei Monate oder ein Jahr umfassen.

In einer Ausführungsform muss sich der Nutzer nach einer Erstinbetriebnahme des Kommunikationssystems gegenüber dem Kommunikationssystem authentifizieren.

Das Authentifizieren des Nutzers nach der Erstinbetriebnahme bewirkt, dass nur ein Nutzer, welcher eine Berechtigung der Nutzung des Kommunikationssystems aufweist, das Kommunikationssystem auch tatsächlich nutzen kann. Die Authentifizierung nach der Erstinbetriebnahme kann beispielsweise durch eine Eingabe eines Passwortes, eines Codes und/oder einer PIN erfolgen. Der Nutzer kann die Berechtigung beispielsweise durch einen Kauf des Kommunikationssystems erwerben.

In einer weiteren Ausführungsform der Erfindung sind die einzelnen physiologischen Parameter bei der Klassifikation des physiologischen Zustandes und/oder die einzelnen Klassifikationsparameter bei der Auswertung der verhaltensbasierten Authentifizierung mit jeweils einem Gewichtungsfaktor gewichtet. Die Gewichtungsfaktoren sind dabei dem jeweiligen physiologischen Parameter bzw. Klassifikationsparameter zugeordnet.

Durch das Verwenden von Gewichtungsfaktoren für die jeweiligen physiologischen Parameter bei der Generierung des Klassifikationsergebnisses bzw. die jeweiligen Klassifikationsparameter bei der Errechnung des Konfidenzwertes ergibt sich in vorteilhafter Weise eine Erhöhung der Genauigkeit des Klassifikationsergebnisses bzw.

Konfidenzwertes bei einer Prüfung gegen ein Prüfungskriterium. Die einzelnen Parameter, können nach Wichtigkeit und/oder Genauigkeit Ihrer Bestimmbarkeit gewichtet werden. Da jeder Nutzer anders ist, spielen auch die einzelnen Parameter unterschiedlich starke Rollen bei der Klassifikation des physiologischen Zustandes und/oder der verhaltensbasierten Authentifizierung des Nutzers.

In einer weiteren Ausführungsform der Erfindung sind die einzelnen Gewichtungsfaktoren der jeweiligen Parameter fest vorgegeben. Fest vorgegeben meint in diesem Zusammenhang, dass die Gewichtungsfaktoren von vornerein bei der Erstinbetriebnahme des Kommunikationssystems festgelegt werden und keine Änderung der Gewichtungsfaktoren während des bestimmungsmäßigen Betriebes des Kommunikationssystems vorgegeben ist.

Durch das Vorgeben der Gewichtungsfaktoren für die Parameter ergibt sich in vorteilhafter Weise ein reduzierter Rechenaufwand, welcher insbesondere für mobile, tragbare Kommunikationssysteme einen niedrigen Batterieverbrauch zufolge hat. Das Kommunikationssystem muss nicht überprüfen, welcher Gewichtungsfaktor für welchen Parameter festzulegen ist, sondern letztendlich nur in seinem Speicher die entsprechenden Gewichtungsfaktoren auslesen.

In einer weiteren Ausführungsform der Erfindung legt der Nutzer in einem Initiierungsprozess die Gewichtungsfaktoren der einzelnen Klassifikationsparameter für die verhaltensbasierte Authentifizierung in dem Kommunikationssystem selbst fest. Die festgelegten Gewichtungsfaktoren werden dann in einer Konfigurationsdatei in dem Speicher des Kommunikationssystems gespeichert.

Durch das Festlegen der Gewichtungsfaktoren durch den registrierten Nutzer selbst ergibt sich in vorteilhafter Weise, dass der registrierte Nutzer selbst bestimmen kann, wie viel sein Verhalten bzw. sein Verhaltensmuster zur verhaltensbasierten Authentifizierung beträgt. Dies erhöht die Freiheit des registrierten Nutzers bei der Konfiguration des Systems, da der Nutzer selbst entscheiden kann, welche Klassifikationsparameter von seinem Verhaltensmuster umfasst werden. Zum Beispiel kann der Nutzer festlegen, dass das Verwenden der Radioanwendung nicht oder nur sehr schwach in die Generierung des Konfidenzwertes miteingehen soll, da er die Radioanwendung üblicherweise nur unregelmäßig nutzt. Der gleiche Nutzer könnte hingegen die Klassifikationsparameter der Positionsbestimmung stärker in die Generierung des Konfidenzwertes miteinfließen lassen, da er einen sehr strukturierten Tagesablauf hat und sich mit hoher Regelmäßigkeit an bestimmten Orten aufhält.

In einer weiteren Ausführungsform der Erfindung sind auf dem Kommunikationssystem mehrere Nutzer registriert und das Klassifikationsergebnis wird für jeden registrierten Nutzer generiert. Ein Nutzererkennungsmodul entscheidet dann, welcher Nutzer gerade aktiv ist, wobei das Nutzererkennungsmodul ebenfalls von dem Prozessor des Kommunikationssystems ausgeführt wird.

In einer weiteren Ausführungsform der Erfindung wird die Identifikation des Nutzers durch das Nutzererkennungsmodul mittels eines Entscheidungsbaums getroffen.

Durch die Möglichkeit, mehrere Nutzer zu identifizieren, ergibt sich in vorteilhafter Weise, dass beispielsweise auch Dienstgeräte bzw. Dienstsysteme, wie mitgeführte Dosimeter in einem durch Radioaktivität belasteten Bereich, die von einem Arbeitgeber an eine Mehrzahl von Mitarbeitern ausgegeben werden, wobei die Mehrzahl von Mitarbeitern, welche das jeweilige Kommunikationssystem abwechselnd benutzen, das Verfahren zur Klassifikation des physiologischen Zustandes anwenden können.

In einer weiteren Ausführungsform der Erfindung ist das Nutzererkennungsmodul so konfiguriert, dass es einen Wechsel des Nutzers anhand von grob- und/oder feinmotorischen Daten erkennt. Das Nutzererkennungsmodul generiert einen zweiten Konfidenzwert, welcher angibt, welcher der registrierten Nutzer gerade der aktuelle Nutzer ist. Der zweite Konfidenzwert wird dann gebildet, wenn das Nutzererkennungsmodul eine Bewegung erkennt, die typisch für einen Nutzerwechsel des Kommunikationssystems ist. Eine typische Bewegung kann dabei das Ab- und Wiederanlegen einer Smartwatch, das Übergeben eines Mobiltelefons oder eine vergleichbare Bewegung umfassen.

In einer weiteren Ausführungsform der Erfindung ist das Nutzererkennungsmodul so konfiguriert, dass es eine zumindest vorübergehende Beendigung der Nutzung des Kommunikationssystems durch den aktuellen Nutzer anhand von grob- und/oder feinmotorischen Daten erkennt. Dazu ist das Nutzererkennungsmodul beispielsweise auf ein Erkennen einer grob- und/oder feinmotorischen Bewegung des Ablegens des Kommunikationssystems konfiguriert. Wird eine solche Beendigung erkannt, werden beispielsweise der bisherige Konfidenzwert verworfen, um sicherzustellen, dass bei einem möglichen Nutzerwechsel ein nicht authentifizierter Nutzer das Kommunikationssystem verwendet. Dieser nicht authentifizierte Nutzer muss sich dann authentifizieren.

In einer weiteren Ausführungsform ist das Kommunikationssystem kommunikativ mit einer Vorrichtung verbunden, wobei das Kommunikationssystem ein Steuersignal erzeugt, welches an die Vorrichtung gesendet wird, um die Vorrichtung anzusteuern, wobei das Steuersignal von dem Klassifikationsergebnis abhängt.

Das Ansteuern einer Vorrichtung in Abhängigkeit von dem Klassifikationsergebnis bewirkt in vorteilhafter Weise eine erhöhte Sicherheit der Vorrichtung. Beispielsweise lässt sich die Vorrichtung nur dann ansteuern, wenn der aktuelle physiologische Zustand eines Nutzers einen bestimmten physiologischen Zustand umfasst oder wenn der aktuelle physiologische Zustand des Nutzers einen bestimmten physiologischen Zustand nicht umfasst. So könnte beispielsweise ein Fahrzeug blockiert werden, wenn der Nutzer betrunken ist oder ihm aus einem anderen Grund schwindelig ist. Ein vorliegendes Schwindelgefühl könnte dabei mittels einer unregelmäßigen grobmotorischen Bewegung beim Gehen erkannt werden. In einem weiteren Beispiel wird ein Flugzeug nur dann betriebsbereit geschaltet, wenn das Klassifikationsergebnis des Piloten einen bestimmten Referenzzustand umfasst.

Die Vorrichtung kann beispielsweise über ein oder mehrere Systeme mit dem Kommunikationssystem verbunden sein. Eine Tür beispielsweise öffnet sich nur dann, falls auch das mindestens eine weitere System ein zweites Steuersignal erzeugt. Das mindestens eine System kann beispielsweise Sensoren umfassen, um selbst Daten des Nutzers zu erfassen und um daraus ein zweites, von dem Klassifikationsergebnis des Kommunikationssystems unabhängiges, Klassifikationsergebnis zu generieren. Ferner kann das mindestens System beispielsweise dazu konfiguriert sein, Teile der durch das Kommunikationssystem erfassten Daten zu empfangen und diese dazu verwenden, das zweite unabhängige Klassifikationsergebnis zu generieren. Das zweite Steuersignal wird beispielsweise nur dann generiert, wenn das unabhängige zweite Klassifikationsergebnis bestimmte Voraussetzungen erfüllt.

Durch die Verbindung des Kommunikationssystems mit der Vorrichtung über mindestens ein weiteres System ergibt sich in Vorteilhafterweise, dass die Vorrichtung nur dann angesteuert wird, wenn eine unabhängige Instanz dies zulässt. Eine solche Anwendung wäre beispielsweise eine Tür, die nur Personal eines Unternehmens, eintreten lässt, welches einen bestimmten physiologischen Zustand aufweist. Eine solche Tür kann beispielsweise einen Reinraum oder ein biologisches Labor schützen, welches nur von Personen betreten werden darf, die einen bestimmten physiologischen Zustand, insbesondere den Referenzzustand aufweisen.

Im Weiteren werden Ausführungsformen der Erfindung mit Bezugnahme auf die Zeichnungen näher erläutert.

Es zeigen:
- Figur 1: einen schematischen Aufbau eines beispielhaften ersten Kommunikationssystems zur verhaltensbasierten Authentifizierung eines Nutzers.
- Figur 2: einen schematischen Ablauf einer verhaltensbasierten Authentifizierung in einem Ablaufdiagramm.
- Figur 3a: Schritte der verhaltensbasierten Authentifizierung in einem schematischen Ablaufdiagramm.

- Figur 3b: Schritte eines Trainings eines Klassifikationsmoduls in einem schematischen Ablaufdiagramm.
- Figur 4: schematische Schritte der verhaltensbasierten Authentifizierung zur Verarbeitung von Daten.
- Figur 5a und 5b: beispielhafte Parameter und ihre Einteilung in Bereiche.
- Figur 6: eine Ausführungsform der Erfindung als schematisches Ablaufdiagramm.
- Figur 7: eine schematische Verbindung zwischen dem Kommunikationssystem und einem elektronischen Heilberufsausweis.

Elemente der nachfolgenden Ausführungsformen, die einander entsprechen, werden mit denselben Bezugszeichen gekennzeichnet.

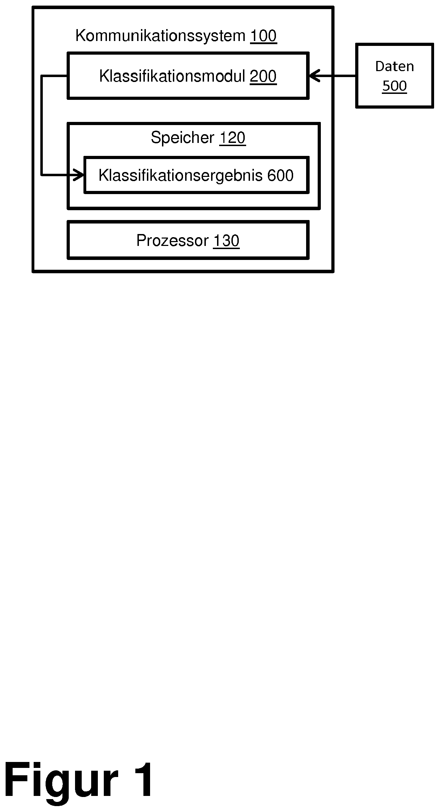

Figur 1 zeigt den schematischen Aufbau eines Kommunikationssystems 100. Das Kommunikationssystem 100 umfasst ein Klassifikationsmodul 200, einen Speicher 120 sowie einen Prozessor 130. Daten 500 werden erfasst und in das Klassifikationsmodul 200 eingegeben. Das Erfassen der Daten 500 unterscheidet sich je nach Art der Daten 500. Die Körpertemperatur wird beispielsweise mittels eines Temperatursensors erfasst, die Pulsfrequenz und der Blutdruck werden beispielsweise mittels eines Drucksensors erfasst.

Grobmotorische Bewegungsdaten werden mittels eines Sensors zur Erfassung von grobmotorischen Bewegungen erfasst. Dieser kann beispielsweise als Gyroskop und/oder Beschleunigungssensor ausgebildet sein. Führt der Nutzer beispielsweise eine grobmotorische Bewegung aus und trägt dabei das mobile Kommunikationssystem 100 bei sich (z. B. in einer Tasche eines Bekleidungsstücks des Nutzers, wie z.B. in seiner Jacken- oder Hosentasche, oder direkt am Körper), so kann der Sensor diese grobmotorische Bewegung in Form von grobmotorischen Bewegungsdaten erfassen, und zwar aufgrund der Bewegung des Kommunikationssystem, die mittelbar (über das Bekleidungsstück) oder unmittelbar (wenn der Nutzer das Kommunikationssystem am Körper trägt) durch die grobmotorische Bewegung des Nutzers verursacht wurde. Die Bewegung des Nutzers kann beispielsweise ein Gehen, ein Joggen, ein Rennen oder eine Bewegung des Armes sein, falls das Kommunikationsgeräts, welches den Sensor aufweist, am Arm befestigt ist.

Unter einer grobmotorischen Bewegung können beispielsweise Bewegungsabläufe wie zum Beispiel Gehen, Joggen, Rennen, Hüpfen, Klettern, Balancieren, Fahrradfahren, Autofahren oder eine Bewegung des Armes etwa beim Trinken, beim Schauen auf eine Armbanduhr oder beim Herausziehen des mobilen, tragbaren Kommunikationssystems 100 aus einer Tasche verstanden werden.

Feinmotorische Bewegungsdaten werden beispielsweise mittels eines Sensors, beispielsweise in Form einer Mensch-Maschine-Schnittstelle, erfasst. Diese kann beispielsweise als Touchscreen und/oder Tastatur oder im Falle des zuvor genannten Zitterns als Gyroskop ausgebildet sein. Der Sensor kann beispielsweise als ein Touchdisplay, eine Tastatur oder eine Kombination aus beidem ausgebildet sein. Die feinmotorische Bewegung des Nutzers kann beispielsweise als Tippgeschwindigkeit, Eingabefrequenz oder Eingabegenauigkeit erfasst werden.

Anwendungsdaten werden mittels entsprechenden Anwendungen und/oder geeigneten Sensoren erfasst. Beispielsweise können durch das Anwendungsnutzungsverhalten selbst Daten 500 generiert werden, ferner können beispielsweise Fotos des Nutzers durch einen Charge-Coupled-Device erfasst und biometrische Daten des Nutzers extrahiert werden.

Beispielsweise könnte ein Foto des Nutzers Daten bezüglich des Erscheinungsbilds der Gesichtshaut und/oder des Schweißflusses umfassen. In einem weiteren Beispiel wird aus der Tippgeschwindigkeit des Nutzers, dem Lidstand der Augen und insbesondere der Kombination aus beidem, ein Wert errechnet, welcher die Müdigkeit des Nutzers quantifiziert und einen Übermüdungszustand des Nutzers identifiziert.

Die Daten 500 werden in das Klassifikationsmodul 200 eingegeben. Das Klassifikationsmodul 200 generiert aus den Daten 500 ein Klassifikationsergebnis 600. Dabei wird das Klassifikationsmodul 200 mittels des Prozessors 130 ausgeführt. Das Klassifikationsergebnis 600 wird in dem Speicher 120 gespeichert und steht dort für einen Ausleseprozess bereit.

Das in Figur 1 dargestellte Kommunikationssystem 100 umfasst ein einzelnes Kommunikationsgerät. Nach Ausführungsformen kann das Kommunikationssystem 100 auch mehrere Geräte umfassen. Das Kommunikationssystem kann beispielsweise, ohne darauf beschränkt zu sein, eine beliebige Kombination aus einer SmartWatch, Smartglasses, einem Smartphone, einem Handheld, einem Tablet, einem Personal Digital Assistant, einem Pager, einem Navigationsgerät, einem Activity Tracker oder einem Gerät zur Erfassung medizinischer Daten umfassen.

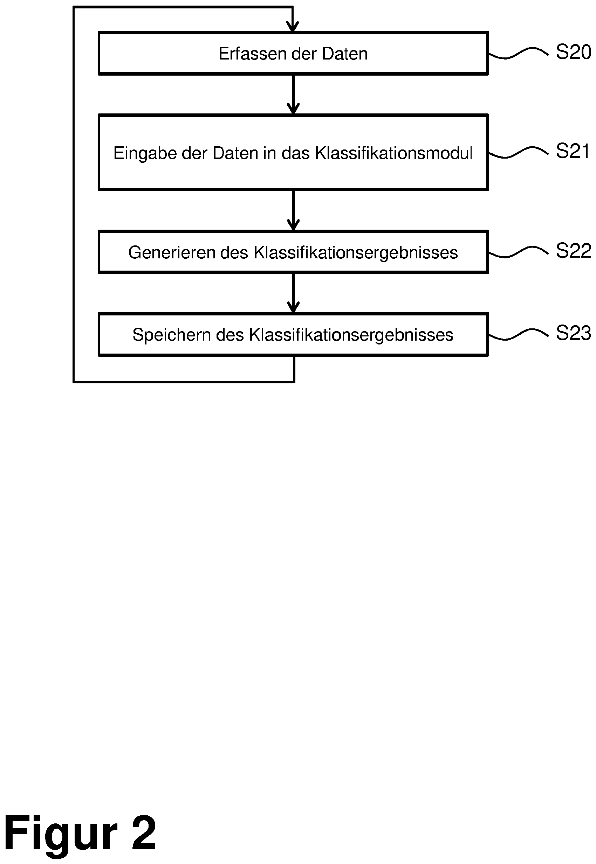

Figur 2 zeigt einen schematischen Ablauf einer verhaltensbasierten Klassifikation eines physiologischen Zustandes in einem Ablaufdiagramm. Die Klassifikation des physiologischen Zustandes wird wiederholt ausgeführt. In Schritt S20 werden die Daten 500 durch einen Sensor und/oder eine Anwendung erfasst. Auf das Erfassen der Daten 500 (S20) folgt eine Eingabe der Daten 500 in das Klassifikationsmodul 200 (S21). Aus den Daten 500 generiert das Klassifikationsmodul 200 ein Klassifikationsergebnis 600 (S22).

In weiteren Ausführungsformen umfasst das Generieren des Klassifikationsergebnisses 600 (S22) ein Vergleichen der Daten 500 mit einem Vergleichsdatensatz. Der Vergleich wird beispielsweise hinsichtlich einer Mehrzahl von Vergleichsparametern durchgeführt. Das Klassifikationsergebnis 600 wird schließlich in den Speicher des Kommunikationssystems 100 gespeichert (S23). Nach Ausführungsformen werden die Schritte S20, S21, S22 und S23 parallel zueinander mit kontinuierlich erfassten Daten ausgeführt, welche kontinuierlich zu einem Klassifikationsergebnis 600 verarbeitet und gespeichert werden.

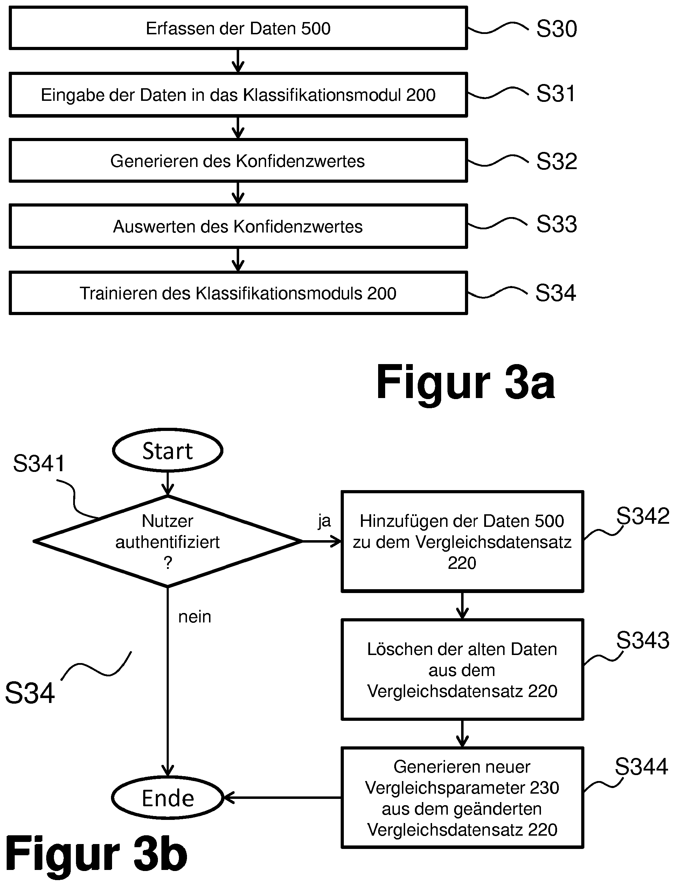

Figur 3a zeigt Schritte einer verhaltensbasierten Authentifizierung gemäß einer Ausführungsform in einem Ablaufdiagramm. Die verhaltensbasierte Authentifizierung wird wiederholt ausgeführt. In Schritt S30 werden die Daten 500 durch einen Sensor und/oder eine Anwendung erfasst. Auf das Erfassen der Daten 500 (S30) folgt eine Eingabe der Daten 500 in das Klassifikationsmodul 200 (S31). Aus den Daten 500 generiert das Klassifikationsmodul 200 einen Konfidenzwert 540 (S32).

In weiteren Ausführungsformen umfasst das Generieren des Konfidenzwerts 540 (S32) ein Vergleichen der Daten 500 mit einem Vergleichsdatensatz. Der Vergleich wird beispielsweise hinsichtlich einer Mehrzahl von Vergleichsparametern durchgeführt. Nach Ausführungsformen laufen die Schritte S30, S31 und S32 parallel zueinander ab, sodass kontinuierlich Daten 500 erfasst werden, welche kontinuierlich zu Konfidenzwerten 540 verarbeitet werden.

In Schritt S33 wird der Konfidenzwert 540 ausgewertet, wobei das Auswerten ein Prüfen umfasst, ob der Konfidenzwert 540 einen vordefinierten Schwellenwert überschreitet. Ist der Schwellenwert bei der Prüfung überschritten, so ist der Nutzer authentifiziert.

Gemäß einer Ausführungsform erfolgt auf eine erfolgreiche Authentifizierung des Nutzers hin ein Trainieren des Klassifikationsmoduls 200 (S34).

Das Trainieren des Klassifikationsmoduls 200 umfasst ein Hinzufügen der in Schritt S30 erfassten Daten 500 zu dem Vergleichsdatensatz, welcher in Schritt S32 genutzt wurde, um den Konfidenzwert 540 zu generieren. Das Trainieren des Klassifikationsmoduls S34 ist detaillierter in Figur 3b dargestellt.

Figur 3b zeigt das Trainieren des Klassifikationsmoduls S34 als Ablaufdiagramm. Zunächst wird überprüft, ob sich der Nutzer erfolgreich authentifizieren konnte S341. Ist die Authentifizierung nicht erfolgreich und der Nutzer somit nicht authentifiziert, so findet kein weiteres Training des Klassifikationsmoduls 200 statt. Konnte der Nutzer authentifiziert werden, so werden zunächst die in S30 erfassten Daten 500 dem Vergleichsdatensatz 220 hinzugefügt S342.

Um das Hinzufügen der Daten 500 zu dem Vergleichsdatensatz 220 zu ermöglichen, weisen die Daten 500 und der Vergleichsdatensatz 220 gemäß einer Ausführungsform eine gleiche Struktur auf. Mit Struktur ist hier das Format der Daten gemeint. Beispielsweise umfassen sowohl die Daten 500 als auch der Vergleichsdatensatz 220 eine Mehrzahl von Werten in Form von Gleitkommazahlen oder ganzen Zahlen. Der Vergleichsdatensatz 220 kann sich gemäß einer weiteren Ausführungsform aus einer Mehrzahl von Daten 500 zusammensetzen, welche zusammen einen Datensatz bilden. Dadurch umfasst der Vergleichsdatensatz 220 automatisch die gleiche Struktur wie die Daten 500, solange sich das Format der erfassten Daten 500 nicht ändert.

Wurden die Daten 500 dem Vergleichsdatensatz 220 hinzugefügt, so wird der Vergleichsdatensatz 220 nach Daten 500 durchsucht, welche ein vordefiniertes Alter erreicht haben. Die gefundenen Daten 500 werden gemäß einer weiteren Ausführungsform aus dem Vergleichsdatensatz 220 gelöscht (S343). Die Reihenfolge der Schritte S342 und S343 ist dabei austauschbar.

Da sich der Vergleichsdatensatz 220 durch das Hinzufügen von Daten 500 und gegebenenfalls durch das Löschen von Daten 500 geändert hat, werden die Vergleichsparameter 230, welche zu dem Generieren des Konfidenzwertes 540 in Schritt S32 beitragen, neu berechnet, um bei einem zukünftigen Authentifizierungsversuch zur Verfügung zu stehen.

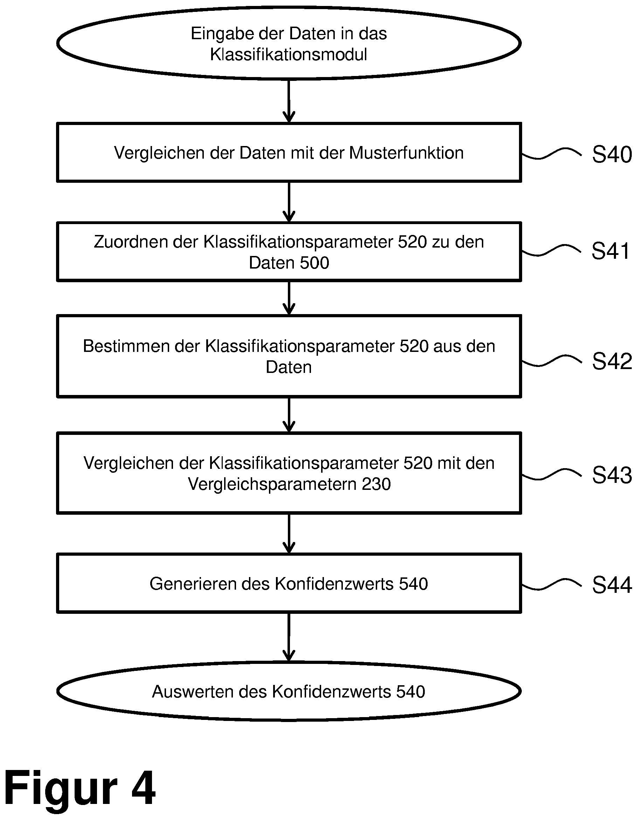

Figur 4 zeigt schematische Schritte der verhaltensbasierten Authentifizierung zur Verarbeitung von Daten gemäß einer Ausführungsform in einem Ablaufdiagramm. Insbesondere Teile des Schritts des Generierens des Konfidenzwerts 540 (vgl. Figur 3a, S32) sind hier dargestellt. Der Prozess startet mit der Eingabe der Daten 500 in das Klassifikationsmodul 200.

Die Daten 500 werden in Schritt S40 zunächst mit einer Musterfunktion 210 verglichen. Das Vergleichen umfasst ein Fitten der Daten 500 unter Verwendung der Musterfunktion 210. War der Fit erfolgreich, werden die Daten 500 Klassifikationsparametern 520 zugeordnet S41. Die Klassifikationsparameter 520 umfassen die Fitparameter des Fits aus S40. War der Fit nicht erfolgreich, so werden die Daten 500 verworfen und es wird kein Konfidenzwert 540 generiert. Ob ein Fit erfolgreich ist oder nicht hängt davon ab, ob die gefundenen Parameter ein vordefiniertes Fehlerkriterium erfüllten oder nicht. Das Fehlerkriterium kann beispielsweise erfüllt sein, wenn ein Fehlerschwellenwert durch die Fehler der Parameter unterschritten wurde oder wenn Parameter gefunden werden konnten, welche den Verlauf der Daten als Fit-Funktion gemäß der Musterfunktion repräsentieren. Die Klassifikationsparameter 520 umfassen gemäß einer weiteren Ausführungsform auch die Fehlerwerte der einzelnen Fitparameter.

Das Bestimmen, welche Parameter für das weitere Verfahren als Klassifikationsparameter verwendet werden bzw. welche Werte diese Klassifikationsparameter aufweisen, ist in Schritt S42 dargestellt. In Schritt S43 werden nun die bestimmten Klassifikationsparameter 520 mit Vergleichsparametern 230 verglichen. Die Vergleichsparameter 230 wurden gemäß einer Ausführungsform analog zu dem Trainingsprozess (dargestellt in Figur 3b) aus älteren Daten berechnet.

Die Differenz zwischen den jeweiligen Klassifikationsparametern 520 und den entsprechenden Vergleichsparametern 230 sagt aus, wie gut die Klassifikationsparameter 520 mit den Vergleichsparametern 230 übereinstimmen. Damit lassen sich die erfassten Daten 500 mit den Daten des Vergleichsdatensatzes vergleichen, was im übertragenen Sinne ein Vergleich des Verhaltens des Nutzers mit dem Nutzer, welcher den Vergleichsdatensatz generierte, also dem registrierten Nutzer, bedeutet.

Auf Basis des Vergleichs der Klassifikationsparameter 520 mit den Vergleichsparametern 230 lässt sich eine Aussage treffen, wie hoch die Wahrscheinlichkeit ist, dass der Nutzer und der registrierte Nutzer dieselbe Person sind. Diese Wahrscheinlichkeit wird durch den Konfidenzwert 540 ausgedrückt, welcher in Schritt S44 generiert wird. Schließlich wird der Konfidenzwert 540 gespeichert.

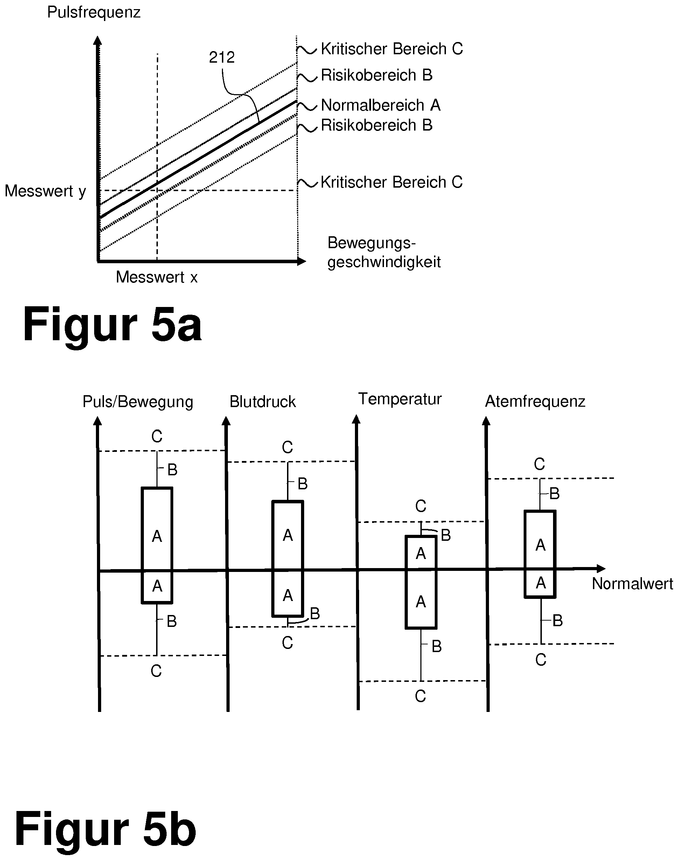

Figur 5a und Figur 5b zeigen beispielhafte Parameter und ihre Einteilung.

In Figur 5a ist schematisch ein Diagramm dargestellt, welches die Pulsfrequenz des Nutzers in Abhängigkeit der Bewegungsgeschwindigkeit darstellt. Der Puls kann beispielsweise mittels eines Drucksensors erfasst werden, welcher an einer geeigneten Stelle getragen wird. Eine solche Stelle kann beispielsweise das Handgelenk, den Hals oder die Schläfe umfassen. Die Bewegungsgeschwindigkeit kann beispielsweise, ohne darauf beschränkt zu sein, aus den physiologischen Parametern von grobmotorischen Bewegungen, insbesondere der Schrittlänge des Nutzers, bestimmt werden oder aus den Positionsdaten des Kommunikationssystems ermittelt werden. Die Positionsdaten umfassen beispielsweise die Positionsdaten eines GPS- oder Galileo-Signals oder werden aus umliegenden WLAN-Signalen ermittelt.

Typischerweise steigt die Pulsfrequenz mit wachsender Bewegungsgeschwindigkeit. In Ausführungsformen wird die Pulsfrequenz im Verhältnis zur Bewegungsintensität betrachtet. Das in Figur 5a dargestellte lineare Verhältnis zwischen Pulsfrequenz und Bewegungsgeschwindigkeit dient hier lediglich der Erläuterung und erhebt keinen Anspruch auf Korrektheit. Das Diagramm dient der Veranschaulichung, dass die Pulsfrequenz mit der Bewegungsgeschwindigkeit ansteigt.

In der Mitte des Diagramms ist der Normalwert 212 der Pulsfrequenz (durchgezogene Linie) dargestellt. Direkt um den Normalwert 212 erstreckt sich der Normalbereich A. Der Normalbereich A richtet sich nach den erfassten Messdaten im Referenzzustand. In einer Ausführungsform wird der Normalbereich A in einem Kalibrierungsverfahren durch den Nutzer bestimmt, indem sich der Nutzer im Referenzzustand befindet und die Daten des entsprechenden Parameters erfasst werden. Wird beispielsweise die Pulsfrequenz im Verhältnis zu einer Bewegung erfasst, wird der Nutzer aufgefordert sich zu bewegen, während der Puls gemessen wird. Die Daten werden erfasst und als Teil des Vergleichsdatensatzes gespeichert. In einer weiteren Ausführungsform ist der Normalbereich A durch Trainingsdatensätze einer Nutzerkohorte bestimmt. Der Parameter, dessen Normalbereich A bestimmt werden soll, wird für eine Mehrzahl von Personen erfasst. Aus den erfassten Daten aller Trainingspersonen wird der Normalbereich A generiert.

Aus den Messwerten des Normalbereichs A wird der Normalwert 212 des Nutzers generiert. Das Generieren des Normalwerts 212 kann beispielsweise die Bildung eines Mittelwerts, eines Medians oder eines Modalwertes umfassen. Die Grenzen des Normalbereichs A können symmetrisch um den Normalwert 212 verteilt sein, wie in Figur 5A dargestellt. Die Grenzen des Normalbereichs A können ferner auch asymmetrisch um den Normalwert 212 verteilt sein (siehe unten, Figur 5b). In einer Ausführungsform werden die Grenzen des Normalbereichs A aus den Fehlerwerten des Normalwerts 212 bestimmt.

Befindet sich ein Parameter außerhalb des Normalbereichs A, so sucht das Klassifikationsmodul nach physiologischen Zuständen, welche eine entsprechende Abweichung des Parameters von dem Normalwert 212 aufweisen. Kann ein solcher physiologischer Zustand gefunden werden, so ist der aktuelle physiologische Zustand des Nutzers klassifiziert.

Ober- und unterhalb des Normalbereichs A liegen der obere und der untere Risikobereich B. Die Grenzen des Risikobereichs B können beispielsweise analog zu den Grenzen des Normalbereichs A bestimmt werden. In einer weiteren Ausführungsform werden die Grenzen des Risikobereichs B manuell festgelegt. Beispielsweise ein Arzt oder ein geschulter Fitnesstrainer legt die Grenzen des Risikobereichs B für einen oder mehrere Parameter fest.

Ein oder mehrere Parameter innerhalb des Risikobereichs B haben jedoch nicht zwangsweise zur Folge, dass das Klassifikationsmodul aus den Daten einen Anormalzustand klassifiziert. Ein Trainingszustand umfasst beispielsweise einen oder mehrere Parameter innerhalb des Risikobereichs B. So sind beispielsweise der Puls und die Atemfrequenz während des Trainings erhöht.

Zusammen mit den Daten einer grobmotorischen Bewegung können die erhöhte Atemfrequenz und die erhöhte Pulsfrequenz als Trainingszustand klassifiziert werden. Sind die Atemfrequenz und die Pulsfrequenz erhöht, ohne dass sich der Nutzer bewegt oder bewegt hat, so deutet dies darauf hin, dass tatsächlich ein negativer physiologischer Zustand vorliegt.

Um dies zu verdeutlichen zeigt Figur 5a die gemessene Pulsfrequenz (waagrechte, gestrichelte Linie, Messwert y). Betrachtet man die gemessene Pulsfrequenz im Vergleich zum Ruhepuls (y-Achsenabschnitt des Normalwertes 212), so liegt die gemessene Pulsfrequenz in dem Risikobereich B. Das Klassifikationsmodul vergleicht die erfasste Pulsfrequenz auch mit dem Normalwert 212 der entsprechenden Bewegungsgeschwindigkeit (senkrechte, gestrichelte Linie, Messwert x). Unter Berücksichtigung der Bewegungsgeschwindigkeit befindet sich die erfasste Pulsfrequenz in dem Normalbereich A.

In Ausführungsformen signalisiert das Kommunikationssystem, falls das Klassifikationsergebnis eine kritische Klassifikation des physiologischen Zustandes umfasst. Eine kritische Klassifikation des physiologischen Zustandes wird dann klassifiziert, wenn der Wert einer der Parameter oberhalb der oberen Grenze des oberen Risikobereichs B oder unterhalb der unteren Grenze des unteren Risikobereichs B liegt. Diese beiden Bereiche sind der kritische Bereich C. Nimmt ein Parameter einen Wert innerhalb des kritischen Bereichs C an, so ist dieser Wert ein Extremwert, welcher eine derartige Abweichung eines Parameters von dem entsprechenden Normalwert ergibt, dass daraus eine ernsthafte Gefahr für den Nutzer zu schließen ist. Eine Pulsfrequenz bei ruhendem und seit einiger Zeit in Ruhe befindlichen Körper von 200 oder 10 Schlägen pro Minute, eine Körpertemperatur von 42,7°C oder 13°C oder aber eine obere Blutdruckgrenze von 220 mmHg stellen beispielhafte Extremwerte dar.

Ferner kann beispielsweise auch eine relativ starke Änderung eines Parameters in einem relativ kurzen Zeitraum eine kritische Klassifikation des physiologischen Zustandes zur Folge haben. Beispielsweise, wenn der Blutdruck innerhalb weniger Sekunden rapide fällt, oder die Pulsfrequenz in einem kurzen Zeitraum, wie etwa wenige Sekunden, auf einen für den Nutzer hohen Wert ansteigt.

Kann ein Extremwert nicht durch einen weiteren Parameter relativiert werden, beispielsweise eine Pulsfrequenz von 180 durch eine intensive Bewegung oder eine Körpertemperatur von 18°C - gemessen am Handgelenk - durch eine Außentemperatur von 4°C, so wird ein kritisches Klassifikationsergebnis generiert.

In der Realität bestimmen viele Parameter gemeinsam den physiologischen Zustand eines Nutzers. Diese Parameter wechselwirken teilweise miteinander, sodass sie allein für sich genommen eine relativ geringe Aussagekraft besitzen. In Ausführungsformen wird deshalb eine Mehrzahl von Parametern als Daten erfasst. Die Parameter selbst und/oder die Kombination der Parameter untereinander werden dann in das Klassifikationsmodul eingegeben. Figur 5b zeigt beispielhaft vier solcher Parameter.

Die einzelnen Parameter bzw. ihre Korrelationen können unterschiedliche Bereiche A, B und C aufweisen, wobei die Grenzen zwischen den einzelnen Bereichen jeweils symmetrisch oder antisymmetrisch sein können. In Ausführungsformen wird das Ergebnis der Einteilung der physiologischen Parameter in die Bereiche A, B und C in das Klassifikationsmodul eingegeben. Beispielsweise würde eine Puls- zu Bewegung-Verhältnis im oberen Normalbereich A, ein Blutdruck im unteren Normalbereich A, eine Körpertemperatur im oberen Risikobereich B und eine Atemfrequenz im oberen Normalbereich A erfasst werden. Durch die Eingabe dieser Werte könnte beispielsweise der physiologische Zustand als "leichtes Fieber" oder "erhöhte Körpertemperatur" klassifiziert werden.

In einer weiteren Ausführungsform werden die Werte der physiologischen Parameter direkt in das Klassifikationsmodul eingegeben. Um die Genauigkeit der Klassifikation zu erhöhen, kann aus den physiologischen Parametern direkt das Klassifikationsergebnis bestimmt werden. Beispielsweise kann ein erster physiologischer Zustand dadurch klassifiziert werden, dass der Wert eines der Parameter in einem kleinen ersten Bereich innerhalb des Risikobereichs B liegt und ein zweiter physiologischer Zustand dadurch klassifiziert werden, dass der Wert in einem kleinen zweiten Bereich innerhalb des Risikobereichs B liegt. Dem ersten und dem zweiten Bereich sind dabei unterschiedliche Grenzen zugeordnet, wobei sich die beiden Bereiche teilweise überlappen können. Die Körpertemperatur kann beispielsweise einen Normalwert von 37°C umfassen. Der Normalbereich sei somit von 36,2°C bis 37,2°C festgelegt. Der obere Risikobereich B reicht beispielsweise von 37,2°C bis 41,5°C. Demzufolge reicht der kritische Bereich C ab 41,5°C aufwärts. Nun können beispielsweise innerhalb des Risikobereichs B ein oder mehrere Bereiche verschiedenen physiologischen Zuständen zugeordnet sein. Bei einer Körpertemperatur von beispielsweise 37,2°C - 38,5°C kann zum Beispiel der physiologische Zustand als "leicht fiebrig" klassifiziert werden. Bei einer Körpertemperatur von 38,5°C - 41,5°C hingegen würde das Klassifikationsergebnis zum Beispiel "hohes Fieber" umfassen.

Überlappen zwei Bereiche einander, zum Beispiel, weil "leicht fiebrig" von 37,2°C bis 39°C definiert ist und "hohes Fieber" von 38° - 41°C reicht, so kann aufgrund des entsprechenden physiologischen Parameters, in diesem Fall eine Körpertemperatur von beispielsweise 38,5°C, keine eindeutige Aussage über den physiologischen Zustand getroffen werden, da der Wert des Parameters in dem Überlappbereich liegt. In Ausführungsformen wird die Mehrdeutigkeit durch eine Betrachtung eines oder mehrerer physiologischer Parameter aufgelöst, sodass eine eindeutige Aussage über den physiologischen Zustand getroffen werden kann. In einer weiteren Ausführungsform wird die Klassifikation des physiologischen Zustandes durch das Bilden einer Mahalanobis-Distanz, einer Euklidischen Distanz und/oder mittels eines Maschinenlernverfahrens bestimmt.

Die Überwachung des Körpers und das permanente Klassifizieren des physiologischen Zustandes kann ferner in vorteilhafter Weise zu einer Früherkennung von Krankheiten, insbesondere schweren Erkrankungen, wie etwa Hirnhautentzündung, Krebs oder ähnlichem beitragen. Die kontinuierlich bzw. regelmäßig erfassten Daten können Indizien für eben diese Krankheiten umfassen und beispielsweise einen Arzt bei einer Voruntersuchung bei der Diagnose unterstützen.

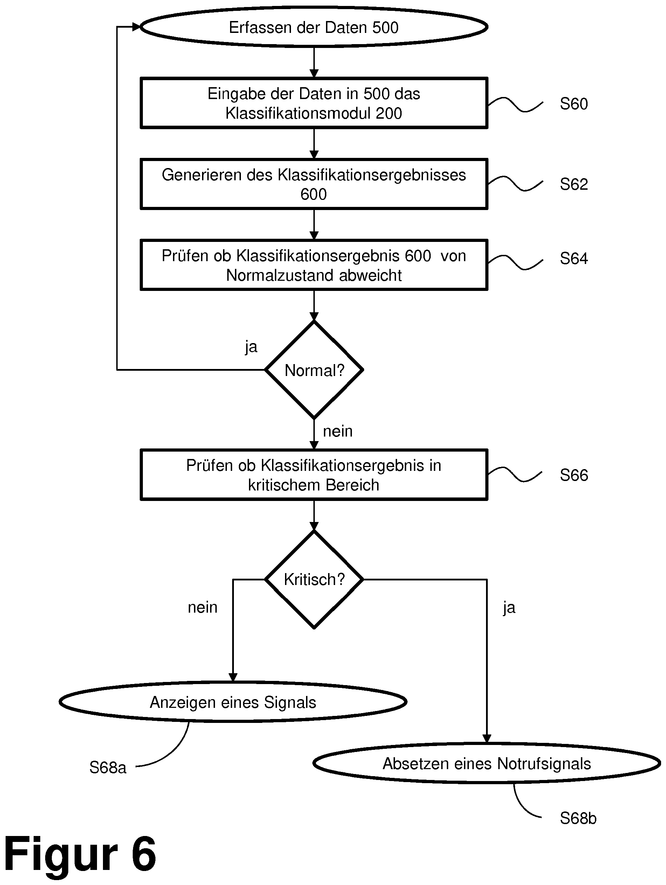

Figur 6 zeigt eine Ausführungsform der Erfindung als Ablaufdiagramm, in der das Kommunikationssystem einen Notruf absetzen kann. Auf das Erfassen der Daten hin werden die Daten in Schritt S60 in das Klassifikationsmodul eingegeben. In Schritt S62 wird aus den Daten das Klassifikationsergebnis generiert. In Schritt S64 wird überprüft, ob das Klassifikationsergebnis von dem Referenzzustand abweicht. Ist dies nicht der Fall, so erfasst das Kommunikationssystem weiter Daten. In einer Ausführungsform werden die Schritte S60, S62 und S64 parallel zueinander ausgeführt.

Weicht das Klassifikationsergebnis von dem Referenzzustand ab, so wird in Schritt S66 geprüft, ob das Klassifikationsergebnis eine kritische Klassifikation des physiologischen Zustandes umfasst. Umfasst das Klassifikationsergebnis eine kritische Klassifikation des physiologischen Zustandes, so zeigt das Kommunikationssystem ein Signal bezüglich des Klassifikationsergebnisses an (S68a). Das Signal kann beispielsweise optisch über einen Bildschirm angezeigt werden. Ferner kann in Ausführungsformen das Signal das Klassifikationsergebnis und/oder Informationen bezüglich des klassifizierten physiologischen Zustandes umfassen. Beispielsweise kann das Signal die aktuelle Pulsfrequenz des Nutzers, einen anderen physiologischen Parameter und/oder Informationen bezüglich der erfassten Daten umfassen.

Umfasst das Klassifikationsergebnis eine kritische Klassifikation des physiologischen Zustandes, wird ein Notruf abgesetzt (S68b). Der Notruf kann beispielsweise ein akustisches Signal, wie etwa einen Alarmton, und/oder ein optisches Signal, wie etwa ein Leuchtsignal, umfassen. Ferner kann der Notruf Informationen bezüglich des Klassifikationsergebnisses, physiologischer Parameter des Nutzers, des Kommunikationssystems, des Nutzers selbst, insbesondere des Standortes und/oder der Identität des Nutzers umfassen. Das Kommunikationssystem kann beispielsweise so konfiguriert sein, dass der Notruf von Umstehenden und/oder in der Nähe befindlichen Personen vernommen werden kann. Ferner kann der Notruf in Ausführungsformen an eine Rettungsstelle und/oder eine vergleichbare Institution gerichtet sein.

Umfasst das Klassifikationsergebnis ein kritisches Klassifikationsergebnis, kann eine Person, die einen Heilberuf ausübt, insbesondere in Notfallsituationen, Zugriff auf die Gesundheitsprofile des Nutzers oder Teile der Gesundheitsprofile erlangen, indem sie sich gegenüber dem Kommunikationssystem authentifiziert. Die Authentifizierung kann beispielsweise mittels eines elektronischen Heilberufsausweis (eH-BA) erfolgen. In Ausführungsformen der Erfindung kann der Inhaber eines eHBAs auch dann Zugriff auf die Gesundheitsprofile des Kommunikationssystems erlangen, wenn das Klassifikationsergebnis kein kritisches Klassifikationsergebnis umfasst, der Nutzer des Kommunikationssystems dem Zugriff jedoch zustimmt. Um einem solchen Zugriff zuzustimmen muss sich der Nutzer beispielsweise gegenüber dem Kommunikationssystem authentifizieren.

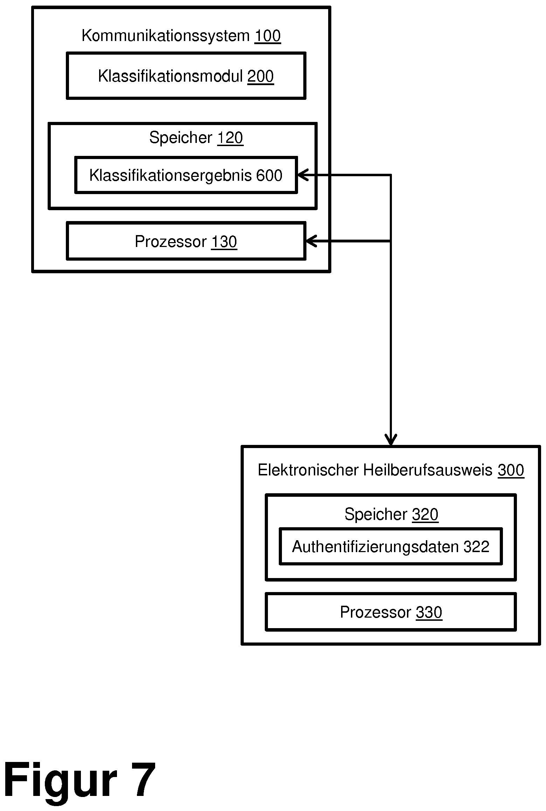

Figur 7 zeigt eine schematische Verbindung zwischen dem Kommunikationssystem 100 und einem eHBA 300. Das Kommunikationssystem 100 umfasst, analog zu Figur 1, das Klassifikationsmodul 200, den Speicher 120 und einen Prozessor 130, wobei in dem Speicher 120 das Klassifikationsergebnis 600 gespeichert ist. Der eHBA umfasst einen Speicher 320 und einen Prozessor 330. In dem Speicher 320 sind Authentifizierungsdaten 322 gespeichert. Die Authentifizierungsdaten authentifizieren den eHBA gegenüber dem Kommunikationssystem. In einer Ausführungsform umfassen die Authentifizierungsdaten ferner Informationen über den Besitzer des eHBAs. In einer weiteren Ausführungsform sind die Authentifizierungsdaten durch eine unabhängige und vertrauenswürdige Instanz, wie beispielsweise ein Trust Center, die Ärztekammer oder ein anderes vertrauenswürdiges Institut, signiert und/oder zertifiziert.

Umfasst das Klassifikationsergebnis ein kritisches Klassifikationsergebnis, so kann gemäß einer Ausführungsform der Besitzer eines eHBAs 300 eine kommunikative Verbindung des eHBAs mit dem Kommunikationssystem 100 herstellen. Dabei wird zunächst geprüft, ob sich der Besitzer des eHBAs gegenüber dem eHBA authentifizieren kann. Die Prüfung erfolgt dabei unter Verwendung der Authentifizierungsdaten 322. Ferner kann die Prüfung beispielsweise das Eingeben einer PIN und/oder eines Passwortes umfassen und/oder ein weiteres Authentifizierungsmittel verwenden. Die Prüfung wird durch den Prozessor 130 und den Prozessor 330 des Kommunikationssystems 100 des eHBAs 300 durchgeführt. Beispielsweise kann die Prüfung mittels eines Challenge-Response-Verfahrens und/oder mittels einer Überprüfung einer Signatur bzw. eines Zertifikats durchgeführt werden.

Konnte sich der Besitzer des eHBAs gegenüber dem Kommunikationssystem authentifizieren, so gewährt das Kommunikationssystem dem Besitzer des eHBAs Zugriff auf das in dem Speicher 120 gespeicherten Klassifikationsergebnis 600. In Ausführungsformen, erlangt der Besitzer des eHBAs Zugriff auf mehrere Klassifikationsergebnisse oder auf Teile davon, je nachdem, welcher Zugriff ihm durch sein eHBA gestattet ist.

### Bezugszeichenliste

- 100:: Kommunikationssystem
- 120:: Speicher
- 130:: Prozessor
- 200:: Klassifikationsmodul
- 210:: Musterfunktion
- 212:: Normalwert
- 220:: Vergleichsdatensatz
- 230:: Vergleichsparameter
- 300:: elektronischer Heilberufsausweis
- 320:: Speicher
- 322:: Authentifizierungsdaten
- 330:: Prozessor
- 500:: Daten
- 520:: Klassifikationsparameter
- 540:: Konfidenzwert
- 600:: Klassifikationsergebnis
- S20:: Erfassen der Daten
- S21:: Eingabe der Daten in das Klassifikationsmodul
- S22:: Generieren des Klassifikationsergebnisses
- S23:: Speichern des Klassifikationsergebnisses
- S30:: Erfassen der Daten
- S31:: Eingabe der Daten in das Klassifikationsmodul
- S32:: Generieren des Konfidenzwertes
- S33:: Speichern des Konfidenzwertes
- S34:: Trainieren des Klassifikationsmoduls
- S341:: Prüfen ob der Nutzer Authentifiziert ist
- S342:: Hinzufügen der Daten zu dem Vergleichsdatensatz
- S343:: Löschen der alten Daten
- S344:: Generieren neuer Vergleichsparameter
- S40:: Vergleichen der Daten
- S41:: Zuordnen der Klassifikationsparameter
- S42:: Bestimmen der Klassifikationsparameter
- S43:: Vergleichen der Klassifikationsparameter
- S44:: Generieren des Konfidenzwerts
- S60:: Eingabe der Daten in das Klassifikationsmodul
- S62:: Generieren des Klassifikationsergebnisses
- S64:: Prüfen des Klassifikationsergebnisses
- S66:: Prüfen des Klassifikationsergebnisses
- S68a:: Anzeigen eines Signals
- S68b:: Absetzen eines Notrufs

## Patentansprüche

1. Elektronisches System zur Klassifikation eines physiologischen Zustandes eines Nutzers,
wobei das System ein mobiles, tragbares Kommunikationssystem (100) umfasst, welches mindestens einen Sensor zur Erfassung von Daten (500), ein Klassifikationsmodul (200), einen Prozessor (130) und einen internen Speicher (120) umfasst,
wobei der Sensor einen internen Sensor des Kommunikationssystems (100) zur Erfassung grobmotorischer Messdaten einer durch eine grobmotorische Bewegung des aktuellen Nutzers des Kommunikationssystems verursachten Bewegung des Kommunikationssystems (100), während der Nutzer das Kommunikationsgerät bei sich trägt, umfasst,
wobei die Daten (500) biometrische Daten des Nutzers umfassen, wobei die biometrischen Daten die grobmotorischen Messdaten der grobmotorischen Bewegung des Nutzers umfassen, wobei die grobmotorische Bewegung eine Bewegung ist, welche eine Bewegung eines oder zweier Beine, eines oder zweier Unterarme, eines oder zweier Arme, des Rumpfes, des Kopfes und/oder der Hüfte des Nutzers umfasst,
wobei das Klassifikationsmodul (200) zur Erkennung einer Mehrzahl von generischen Zuständen einer Mehrzahl von physiologischen Parametern mit Hilfe von Trainingsdatensätzen einer Nutzerkohorte trainiert ist, wobei das Klassifikationsmodul (200) durch den Prozessor (130) des Kommunikationssystems (100) ausgeführt wird,
wobei das System die folgenden Schritte ausführt:
• Erfassen der Daten (500) durch den mindestens einen Sensor,
• Eingeben der Daten (500) in das Klassifikationsmodul (200),
• Generieren eines Klassifikationsergebnisses (600) des physiologischen Zustandes des Nutzers aus den Daten (500) durch das Klassifikationsmodul (200),
• Speichern des Klassifikationsergebnisses (600) des physiologischen Zustandes des Nutzers in dem Speicher (120) des Kommunikationssystems (100),
wobei das Klassifikationsergebnis (600) des physiologischen Zustandes des Nutzers ein aktuelles Gesundheitsprofil des Nutzers umfasst,
wobei das Gesundheitsprofil eine Klassifikation des physiologischen Zustandes des Nutzers hinsichtlich der Mehrzahl von generischen Zuständen der Mehrzahl von physiologischen Parametern umfasst,
wobei auf die Eingabe der Daten (500) in das Klassifikationsmodul (200) hin eine verhaltensbasierte Authentifizierung des Nutzers durch ein Grobmotorikklassifikationsmodul des mobilen, tragbaren Kommunikationssystems durchgeführt wird, wobei das Grobmotorikklassifikationsmodul für eine Klassifikation der grobmotorischen Messdaten konfiguriert und auf ein Erkennen der grobmotorischen Bewegung eines registrierten Nutzers trainiert ist,
wobei in dem Speicher (120) des Kommunikationssystems (100) mindestens eine Musterfunktion (210) und ein Vergleichsdatensatz (220) gespeichert sind,
wobei die verhaltensbasierte Authentifizierung des Nutzers umfasst:
- Wiederholtes Ausführen der Schritte:
o Erfassen der grobmotorischen Messdaten durch den internen Sensor des mobilen, tragbaren Kommunikationssystems,
∘ Eingabe der grobmotorischen Messdaten in das Grobmotorikklassifikationsmodul,
o Generieren eines Klassifikationsergebnisses des Grobmotorikklassifikationsmoduls durch das Grobmotorikklassifikationsmodul, ob der Nutzer ein in dem mobilen, tragbaren Kommunikationssystem registrierte Nutzer ist,
o Speichern des Klassifikationsergebnisses des Grobmotorikklassifikationsmoduls in dem Speicher des mobilen, tragbaren Kommunikationssystems,
- auf eine Authentifizierungsanfrage hin, Zugreifen auf den Speicher des mobilen, tragbaren Kommunikationssystems, um mindestens eines der gespeicherten Klassifikationsergebnisse des Grobmotorikklassifikationsmoduls aus dem Speicher auszulesen,
- Auslesen und Auswerten des mindestens einen Klassifikationsergebnisses des Grobmotorikklassifikationsmoduls gemäß einem vorgegebenen Prüfungskriterium,
- Erzeugen eines Authentifizierungssignals, falls das Prüfungskriterium erfüllt ist, wobei das Authentifizierungssignal eine erfolgreiche Authentifizierung des aktuellen Nutzers signalisiert.
wobei das Generieren des Klassifikationsergebnisses des Grobmotorikklassifikationsmoduls durch das Grobmotorikklassifikationsmodul folgende Schritte umfasst:
• Vergleichen der grobmotorischen Messdaten der Daten (500) mit der mindestens einen Musterfunktion (210),
• Generieren von Klassifikationsparametern (520) aus dem Vergleich der grobmotorischen Messdaten der Daten (500) mit der Musterfunktion (210),
• Generieren von Vergleichsparametern (230) aus dem Vergleichsdatensatz (220),
• Vergleichen der Klassifikationsparameter (520) mit den Vergleichsparametern (230),
• Generieren eines ersten Konfidenzwertes (540) aus dem Vergleich der Klassifikationsparameter (520) mit den Vergleichsparametern (230),
wobei der erste Konfidenzwert (540) eine Wahrscheinlichkeit umfasst, mit der der Nutzer als der in dem Kommunikationssystem (100) registrierte Nutzer identifiziert wird, wobei das auf den grobmotorischen Messdaten beruhende Klassifikationsergebnis des Grobmotorikklassifikationsmoduls aus dem ersten Konfidenzwert generiert wird,
wobei das Generieren des Klassifikationsergebnisses (600) des physiologischen Zustandes des Nutzers nur dann durchgeführt wird, falls der erste Konfidenzwert (540) einen Schwellenwert überschreitet.

2. Elektronisches System nach Anspruch 1, wobei die biometrischen Daten ferner Daten einer feinmotorischen Bewegung des Nutzers umfassen,
wobei die Mehrzahl von physiologischen Parametern Parameter einer feinmotorischen Bewegung umfassen, und/oder
wobei das Kommunikationssystem (100) Anwendungen umfasst,
wobei die Daten (500) ferner Anwendungsdaten des Nutzers umfassen,
wobei aus den Anwendungsdaten Anpassungsparameter bestimmt werden, welche externe Einflussfaktoren der biometrischen Daten beschreiben, wobei die Anpassungsparameter bei der Generierung des Klassifikationsergebnisses (600) des physiologischen Zustandes des Nutzers zur Anpassung der Klassifikation an die Einflussfaktoren verwendet werden.

3. Elektronisches System nach einem der vorherigen Ansprüche,
wobei das Generieren des Klassifikationsergebnisses (600) des physiologischen Zustandes des Nutzers ein Berechnen einer Mehrzahl nutzerspezifischer Parameter unter Verwendung der biometrischen Daten umfasst, wobei die Mehrzahl nutzerspezifischer Parameter mit der Mehrzahl physiologischer Parameter der generischen Zustände verglichen wird, wobei durch den Vergleich der Parameter mit einander der physiologische Zustand des Nutzers hinsichtlich der Mehrzahl von generischen Zuständen von physiologischen Parametern klassifiziert wird.

4. Elektronisches System nach einem der vorherigen Ansprüche, wobei das Kommunikationssystem (100) dem Nutzer mittels eines Signals signalisiert, falls die Klassifikation des physiologischen Zustandes einen physiologischen Zustand außerhalb eines Normalbereichs umfasst.

5. Elektronisches System nach einem der vorherigen Ansprüche, wobei das Kommunikationssystem (100) dazu konfiguriert ist einen Notruf abzusetzen, falls das Klassifikationsergebnis (600) des physiologischen Zustandes des Nutzers eine kritische Klassifikation des physiologischen Zustandes des Nutzers umfasst.

6. Elektronisches System nach einem der vorherigen Ansprüche, wobei das Kommunikationssystem (100) eine Schnittstelle aufweist, wobei das Kommunikationssystem (100) dazu konfiguriert ist über die Schnittstelle mit einem elektronischen Heilberufsausweis (300) zu kommunizieren, wobei ein Besitzer eines elektronischen Heilberufsausweises (300) Zugriff auf die gespeicherten Gesundheitsprofile des Nutzers erlangt, wobei das Erlangen des Zugriffs die folgenden Schritte umfasst:
• Aufbauen einer kryptographisch gesicherten Verbindung zwischen dem elektronischen Heilberufsausweis und dem Kommunikationssystem,
• Authentifizieren des Besitzers des Heilberufsausweises (300),
• Prüfen, ob ein kritischer physiologischer Zustand des Nutzers vorliegt,
• Gewähren des Zugriffs auf die gespeicherten Gesundheitsprofile des Nutzers durch das Kommunikationssystem, falls das Klassifikationsergebnis (600) des physiologischen Zustandes des Nutzers einen kritischen physiologischen Zustand des Nutzers umfasst.

7. Elektronisches System nach einem der vorherigen Ansprüche, wobei das Kommunikationssystem (100) ein Ausgabemittel aufweist, wobei das Ausgabemittel medizinische Informationen bezüglich des Nutzers, Informationen bezüglich des Klassifikationsergebnisses (600) des physiologischen Zustandes des Nutzers und/oder Sofortmaßnahmen für einen Ersthelfer anzeigt, falls das Klassifikationsergebnis (600) des physiologischen Zustandes des Nutzers einen kritischen physiologischen Zustand des Nutzers umfasst.

8. Elektronisches System nach einem der vorherigen Ansprüche, wobei die verhaltensbasierte Authentifizierung ferner ein Trainieren des Klassifikationsmoduls (200) umfasst, sofern die verhaltensbasierte Authentifizierung erfolgreich war,
wobei das Trainieren des Klassifikationsmoduls (200) ein Hinzufügen der Daten (500) zu dem Vergleichsdatensatz (220) und ein Entfernen von Daten (500), welche älter als ein Schwellenalter sind, umfasst, und/oder
wobei die Mehrzahl von generischen Zuständen der Mehrzahl von physiologischen Parametern eine Auswahl von für die Authentifizierung zulässiger Zustände umfasst, wobei eine erfolgreiche Authentifizierung des Nutzers voraussetzt, dass der physiologische Zustand des Nutzers als ein zulässiger Zustand klassifiziert wird.

9. Elektronisches System nach einem der vorherigen Ansprüche, wobei auf dem Kommunikationssystem (100) mehrere Nutzer registriert sind und ein Klassifikationsergebnis (600) des physiologischen Zustandes des Nutzers für jeden Nutzer generiert wird, wobei jedem Nutzer ein Gesundheitsprofil zugeordnet ist, wobei das Kommunikationssystem (100) ein Nutzererkennungsmodul (200) aufweist, wobei das Nutzererkennungsmodul (200) dazu konfiguriert ist einen Nutzer als einen der registrierten Nutzer zu identifizieren, wobei das Nutzererkennungsmodul (200) von dem Prozessor (130) des Kommunikationssystems (100) ausgeführt wird,
wobei das Nutzererkennungsmodul konfiguriert ist einen Nutzerwechsel anhand der Daten (500) zu erkennen,
wobei das Nutzererkennungsmodul auf ein Erkennen einer grob- und/oder feinmotorischen Bewegung des Ab- und/oder Anlegens des Kommunikationssystems (100) konfiguriert und auf ein Erkennen von nutzerspezifischen Bewegungsmustern in den Daten (500) trainiert ist, wobei die verhaltensbasierte Authentifizierung ferner umfasst:
• Eingabe der Daten (500) in das Nutzererkennungsmodul,
• Generieren mindestens eines zweiten Konfidenzwertes (540) durch das Nutzererkennungsmodul,
• Auswerten des mindestens einen zweiten Konfidenzwertes (540), um zu überprüfen, ob ein Nutzerwechsel stattgefunden hat,
• Speichern des mindestens einen zweiten Konfidenzwertes (540) in dem Speicher (120) des Kommunikationssystems (100),
• Zuordnen der Daten (500) zu einem der Gesundheitsprofile entsprechend dem zweiten Konfidenzwert (540).

10. Elektronisches System nach einem der vorherigen Ansprüche, wobei das Kommunikationssystem (100) kommunikativ mit einer Vorrichtung verbunden ist, wobei das Kommunikationssystem (100) ein Steuersignal erzeugt, welches an die Vorrichtung gesendet wird, um die Vorrichtung anzusteuern, wobei das Steuersignal von dem Klassifikationsergebnis (600) des physiologischen Zustandes des Nutzers abhängt.

11. Verfahren zur Klassifikation eines physiologischen Zustandes eines Nutzers eines mobilen, tragbaren Kommunikationssystems (100),
welches mindestens einen Sensor zur Erfassung von Daten (500), ein Klassifikationsmodul (200), einen Prozessor (130) und einen internen Speicher (120) umfasst,
wobei der Sensor einen internen Sensor des Kommunikationssystems (100) zur Erfassung einer durch eine grobmotorische Bewegung des aktuellen Nutzers des Kommunikationssystems verursachten Bewegung des Kommunikationssystems (100), während der Nutzer das Kommunikationsgerät bei sich trägt, umfasst,
wobei die Daten (500) biometrische Daten des Nutzers umfassen, wobei die biometrischen Daten die grobmotorischen Messdaten der grobmotorischen Bewegung des Nutzers umfassen, wobei die grobmotorische Bewegung eine Bewegung ist, welche eine Bewegung eines oder zweier Beine, eines oder zweier Unterarme, eines oder zweier Arme, des Rumpfes, des Kopfes und/oder der Hüfte des Nutzers umfasst,
wobei das Klassifikationsmodul (200) zur Erkennung einer Mehrzahl von generischen Zuständen einer Mehrzahl von physiologischen Parametern mit Hilfe von Trainingsdatensätzen einer Nutzerkohorte trainiert ist, wobei das Klassifikationsmodul (200) durch den Prozessor (130) des Kommunikationssystems (100) ausgeführt wird,
wobei das Verfahren die folgenden Schritte umfasst:
• Erfassen der Daten (500) durch den mindestens einen Sensor,
• Eingeben der Daten (500) in das Klassifikationsmodul (200),
• Generieren eines Klassifikationsergebnisses (600) des physiologischen Zustandes des Nutzers aus den Daten (500) durch das Klassifikationsmodul (200),
• Speichern des Klassifikationsergebnisses (600) des physiologischen Zustandes des Nutzers in dem Speicher (120) des Kommunikationssystems (100),
wobei das Klassifikationsergebnis (600) des physiologischen Zustandes des Nutzers ein aktuelles Gesundheitsprofil des Nutzers umfasst,
wobei das Gesundheitsprofil eine Klassifikation des physiologischen Zustandes des Nutzers hinsichtlich der Mehrzahl von generischen Zuständen der Mehrzahl von physiologischen Parametern umfasst,
wobei auf die Eingabe der Daten (500) in das Klassifikationsmodul (200) hin eine verhaltensbasierte Authentifizierung des Nutzers durch ein Grobmotorikklassifikationsmodul des mobilen, tragbaren Kommunikationssystems durchgeführt wird, wobei das Grobmotorikklassifikationsmodul für eine Klassifikation der grobmotorischen Messdaten konfiguriert und auf ein Erkennen der grobmotorischen Bewegung eines registrierten Nutzers trainiert ist,
wobei in dem Speicher (120) des Kommunikationssystems (100) mindestens eine Musterfunktion (210) und ein Vergleichsdatensatz (220) gespeichert sind,
wobei die verhaltensbasierte Authentifizierung des Nutzers umfasst:
- Wiederholtes Ausführen der Schritte:
∘ Erfassen der grobmotorischen Messdaten durch den internen Sensor des mobilen, tragbaren Kommunikationssystems,
∘ Eingabe der grobmotorischen Messdaten in das Grobmotorikklassifikationsmodul,
∘ Generieren eines Klassifikationsergebnisses des Grobmotorikklassifikationsmoduls durch das Grobmotorikklassifikationsmodul, ob der Nutzer ein in dem mobilen, tragbaren Kommunikationssystem registrierte Nutzer ist,
∘ Speichern des Klassifikationsergebnisses des Grobmotorikklassifikationsmoduls in dem Speicher des mobilen, tragbaren Kommunikationssystems,
- auf eine Authentifizierungsanfrage hin, Zugreifen auf den Speicher des mobilen, tragbaren Kommunikationssystems, um mindestens eines der gespeicherten Klassifikationsergebnisse des Grobmotorikklassifikationsmoduls aus dem Speicher auszulesen,
- Auslesen und Auswerten des mindestens einen Klassifikationsergebnisses des Grobmotorikklassifikationsmoduls gemäß einem vorgegebenen Prüfungskriterium,
- Erzeugen eines Authentifizierungssignals, falls das Prüfungskriterium erfüllt ist, wobei das Authentifizierungssignal eine erfolgreiche Authentifizierung des aktuellen Nutzers signalisiert.
wobei das Generieren des Klassifikationsergebnisses des Grobmotorikklassifikationsmoduls durch das Grobmotorikklassifikationsmodul folgende Schritte umfasst:
• Vergleichen der grobmotorischen Messdaten der Daten (500) mit der mindestens einen Musterfunktion (210),
• Generieren von Klassifikationsparametern (520) aus dem Vergleich der grobmotorischen Messdaten der Daten (500) mit der Musterfunktion (210),
• Generieren von Vergleichsparametern (230) aus dem Vergleichsdatensatz (220),
• Vergleichen der Klassifikationsparameter (520) mit den Vergleichsparametern (230),
• Generieren eines ersten Konfidenzwertes (540) aus dem Vergleich der Klassifikationsparameter (520) mit den Vergleichsparametern (230),
wobei der erste Konfidenzwert (540) eine Wahrscheinlichkeit umfasst, mit der der Nutzer als der in dem Kommunikationssystem (100) registrierte Nutzer identifiziert wird, wobei das auf den grobmotorischen Messdaten beruhenden Klassifikationsergebnisses des Grobmotorikklassifikationsmoduls aus dem ersten Konfidenzwert generiert wird,
wobei das Generieren des Klassifikationsergebnisses (600) des physiologischen Zustandes des Nutzers nur dann durchgeführt wird, falls der erste Konfidenzwert (540) einen Schwellenwert überschreitet.

12. Verfahren nach Anspruch 11, wobei die biometrischen Daten ferner Daten einer feinmotorischen Bewegung des Nutzers umfassen, wobei die Mehrzahl von physiologischen Parametern Parameter einer feinmotorischen Bewegung umfassen, und/oder
wobei das Kommunikationssystem (100) Anwendungen umfasst, wobei die Daten (500) ferner Anwendungsdaten des Nutzers umfassen, wobei aus den Anwendungsdaten Anpassungsparameter bestimmt werden, welche externe Einflussfaktoren der biometrischen Daten beschreiben, wobei die Anpassungsparameter bei der Generierung des Klassifikationsergebnisses (600) des physiologischen Zustandes des Nutzers zur Anpassung der Klassifikation an die Einflussfaktoren verwendet werden.

13. Verfahren nach einem der Ansprüche 11 bis 12, wobei das Generieren des Klassifikationsergebnisses (600) des physiologischen Zustandes des Nutzers ein Berechnen einer Mehrzahl nutzerspezifischer Parameter unter Verwendung der biometrischen Daten umfasst, wobei die Mehrzahl nutzerspezifischer Parameter mit der Mehrzahl physiologischer Parameter der generischen Zustände verglichen wird, wobei durch den Vergleich der Parameter mit einander der physiologische Zustand des Nutzers hinsichtlich der Mehrzahl von generischen Zuständen von physiologischen Parametern klassifiziert wird.

14. Verfahren nach einem der Ansprüche 11 bis 13, wobei das Kommunikationssystem (100) dem Nutzer mittels eines Signals signalisiert, falls die Klassifikation des physiologischen Zustandes einen physiologischen Zustand außerhalb eines Normalbereichs umfasst, und/oder
wobei das Kommunikationssystem (100) dazu konfiguriert ist einen Notruf abzusetzen, falls das Klassifikationsergebnis (600) des physiologischen Zustandes des Nutzers eine kritische Klassifikation des physiologischen Zustandes des Nutzers umfasst, und/oder
wobei die Mehrzahl von generischen Zuständen der Mehrzahl von physiologischen Parametern eine Auswahl von für eine Authentifizierung des Nutzers zulässiger Zustände umfasst, wobei eine erfolgreiche Authentifizierung des Nutzers voraussetzt, dass der physiologische Zustand des Nutzers als ein zulässiger Zustand klassifiziert wird.

## Claims

1. An electronic system for classifying a physiological state of a user,
wherein the system comprises a mobile, wearable communication system (100), which comprises at least one sensor for capturing data (500), a classification module (200, a processor (130) and an internal memory (120),
wherein the sensor comprises an internal sensor of the communication system (100) for capturing gross motor measurement data of a movement of the communication system (100), caused by a gross motor movement of the current user of the communication system, whilst the user is wearing the communication device,
wherein the data (500) comprise biometric data of the user, wherein the biometric data comprise the gross motor measurement data of the gross motor movement of the user, wherein the gross motor movement is a movement which comprises a movement of one or two legs, one or two lower arms, one or two arms, the torso, the head and/or the hip of the user,
wherein the classification module (200) is trained to identify a plurality of generic states of a plurality of physiological parameters with the aid of training datasets of a user group, wherein the classification module (200) is run by the processor (130) of the communication system (100),
wherein the system carries out the following steps:
• capturing the data (500) by the at least one sensor,
• inputting the data (500) into the classification module (200),
• generating a classification result (600) of the physiological state of the user from the data (500) by the classification module (200),
• storing the classification result (600) of the physiological state of the user in the memory (120) of the communication system (100),
wherein the classification result (600) of the physiological state of the user comprises a current health profile of the user,
wherein the health profile comprises a classification of the physiological state of the user in respect of the plurality of generic states of the plurality of physiological parameters,
wherein, in response to the input of the data (500) into the classification module (200), a behaviour-based authentication of the user is carried out by a gross motor classification module of the mobile, wearable communication system, wherein the gross motor classification module is configured for a classification of the gross motor measurement data and is trained to identify the gross motor movement of a registered user,
wherein at least one pattern function (210) and a comparative dataset (220) are stored in the memory (120) of the communication system (100),
wherein the behaviour-based authentication of the user comprises:
- repeatedly carrying out the steps of:
∘ capturing the gross motor measurement data by the internal sensor of the mobile, wearable communication system,
∘ inputting the gross motor measurement data into the gross motor classification module,
∘ generating a classification result of the gross motor classification module by the gross motor classification module, if the user is a user registered in the mobile, wearable communication system,
∘ storing the classification result of the gross motor classification module in the memory of the mobile, wearable communication system,
- in response to an authentication request, accessing the memory of the mobile, wearable communication system in order to read at least one of the stored classification results of the gross motor classification module from the memory,
- reading and evaluating the at least one classification result of the gross motor classification module according to a predefined assessment criterion,
- generating an authentication signal if the assessment criterion is satisfied, wherein the authentication signal signals a successful authentication of the current user,
wherein the generation of the classification result of the gross motor classification module by the gross motor classification module comprises the following steps:
• comparing the gross motor measurement data of the data (500) with the at least one pattern function (210),
• generating classification parameters (520) from the comparison of the gross motor measurement data of the data (500) with the pattern function (210),
• generating comparison parameters (230) from the comparison dataset (220),
• comparing the classification parameter (520) with the comparison parameters (230),
• generating a first confidence value (540) from the comparison of the classification parameter (520) with the comparison parameters (230),
wherein the first confidence value (540) comprises a likelihood with which the user will be identified as the user registered in the communication system (100), wherein the classification result of the gross motor classification module based on the gross motor measurement data is generated from the first confidence value,
wherein the generation of the classification result (600) of the physiological state of the user is carried out only if the first confidence value (540) exceeds a threshold value.

2. The electronic system according to claim 1, wherein the biometric data also comprise data relating to a fine motor movement of the user,
wherein the plurality of physiological parameters comprise parameters of a fine motor movement, and/or
wherein the communication system (100) comprises applications,
wherein the data (500) also comprise application data of the user,
wherein adjustment parameters, which describe external influencing factors of the biometric data, are determined from the application data, wherein the adjustment parameters are used in the generation of the classification result (600) of the physiological state of the user to adjust the classification to the influencing factors.

3. The electronic system according to any one of the preceding claims,
wherein the generation of the classification result (600) of the physiological state of the user comprises a calculation of a plurality of user-specific parameters with use of the biometric data, wherein the plurality of user-specific parameters is compared with the plurality of physiological parameters of the generic states, wherein, by way of the comparison of the parameters with one another, the physiological state of the user is classified in respect of the plurality of generic states of physiological parameters.

4. The electronic system according to any one of the preceding claims, wherein the communication system (100) uses a signal to signal to the user if the classification of the physiological state comprises a physiological state outside a normal range.

5. The electronic system according to any one of the preceding claims, wherein the communication system (100) is configured to make an emergency call if the classification result (600) of the physiological state of the user comprises a critical classification of the physiological state of the user.

6. The electronic system according to any one of the preceding claims, wherein the communication system (100) has an interface, wherein the communication system (100) is configured to communicate with an electronic health professional card (300) via the interface, wherein a user of an electronic health professional card (300) is granted access to the stored health profiles of the user, wherein the granting of access comprises the following steps:
• establishing a cryptographically secured connection between the electronic health professional card and the communication system,
• authenticating the owner of the health professional card (300),
• checking whether the user is in a critical physiological state,
• granting access to the stored health profiles of the user by the communication system if the classification result (600) of the physiological state of the user comprises a critical physiological state of the user.

7. The electronic system according to any one of the preceding claims, wherein the communication system (100) has an output means, wherein the output means displays medical information relating to the user, information relating to the classification result (600) of the physiological state of the user and/or emergency measures for a first-aider if the classification result (600) of the physiological state of the user comprises a critical physiological state of the user.

8. The electronic system according to any one of the preceding claims, wherein the behaviour-based authentication also comprises a training of the classification module (200) if the behaviour-based authentication was successful,
wherein the training of the classification module (200) comprises an addition of the data (500) to the comparison dataset (220) and a removal of data (500) which are older than a threshold age, and/or
wherein the plurality of generic states of the plurality of physiological parameters comprises a selection of states admissible for the authentication, wherein a successful authentication of the user presupposes that the physiological state of the user is classified as an admissible state.

9. The electronic system according to any one of the preceding claims, wherein a plurality of users are registered on the communication system (100) and a classification result (600) of the physiological state of the user is generated for each user, wherein each user is assigned a health profile, wherein the communication system (100) has a user identification module (200), wherein the user identification module (200) is configured to identify a user as one of the registered users, wherein the user identification module (200) is run by the processor (130) of the communication system (100),
wherein the user identification module is configured to identify a change of user on the basis of the data (500),
wherein the user identification module is configured to identify a gross and/or fine motor movement when the communication system (100) is put on and/or taken off and to identify user-specific movement patterns in the data (500), wherein the behaviour-based authentication further comprises:
• inputting the data (500) into the user identification module,
• generating at least one second confidence value (540) by the user identification module,
• evaluating the at least one second confidence value (540) in order to check whether a change of user has taken place,
• storing the at least one second confidence value (540) in the memory (120) of the communication system (100),
• assigning the data (500) to one of the health profiles in accordance with the second confidence value (540).

10. The electronic system according to any one of the preceding claims, wherein the communication system (100) is communicatively connected to a device, wherein the communication system (100) generates a control signal which is sent to the device in order to actuate the device, wherein the control signal is dependent on the classification result (600) of the physiological state of the user.

11. A method for classifying a physiological state of a user of a mobile, wearable communication system (100),
which comprises at least one sensor for capturing data (500), a classification module (200), a processor (130) and an internal memory (120),
wherein the sensor comprises an internal sensor of the communication system (100) for capturing a movement of the communication system (100), caused by a gross motor movement of the current user of the communication system, whilst the user is wearing the communication device,
wherein the data (500) comprise biometric data of the user, wherein the biometric data comprise the gross motor measurement data of the gross motor movement of the user, wherein the gross motor movement is a movement which comprises a movement of one or two legs, one or two forearms, one or two arms, the torso, the head and/or the hip of the user,
wherein the classification module (200) is trained to identify a plurality of generic states of a plurality of physiological parameters with the aid of training datasets of a user group, wherein the classification module (200) is run by the processor (130) of the communication system (100),
wherein the method comprises the following steps:
• capturing the data (500) by the at least one sensor,
• inputting the data (500) into the classification module (200),
• generating a classification result (600) of the physiological state of the user from the data (500) by the classification module (200),
• storing the classification result (600) of the physiological state of the user in the memory (120) of the communication system (100),
wherein the classification result (600) of the physiological state of the user comprises a current health profile of the user,
wherein the health profile comprises a classification of the physiological state of the user in respect of the plurality of generic states of the plurality of physiological parameters,
wherein, in response to the input of the data (500) into the classification module (200), a behaviour-based authentication of the user is carried out by a gross motor classification module of the mobile, wearable communication system, wherein the gross motor classification module is configured for a classification of the gross motor measurement data and is trained to identify the gross motor movement of a registered user,
wherein at least one pattern function (210) and a comparative dataset (220) are stored in the memory (120) of the communication system (100),
wherein the behaviour-based authentication of the user comprises:
- repeatedly carrying out the steps of:
∘ capturing the gross motor measurement data by the internal sensor of the mobile, wearable communication system,
∘ inputting the gross motor measurement data into the gross motor classification module,
∘ generating a classification result of the gross motor classification module by the gross motor classification module, if the user is a user registered in the mobile, wearable communication system,
∘ storing the classification result of the gross motor classification module in the memory of the mobile, wearable communication system,
- in response to an authentication request, accessing the memory of the mobile, wearable communication system in order to read at least one of the stored classification results of the gross motor classification module from the memory,
- reading and evaluating the at least one classification result of the gross motor classification module according to a predefined assessment criterion,
- generating an authentication signal if the assessment criterion is satisfied, wherein the authentication signal signals a successful authentication of the current user,
wherein the generation of the classification result of the gross motor classification module by the gross motor classification module comprises the following steps:
• comparing the gross motor measurement data of the data (500) with the at least one pattern function (210),
• generating classification parameters (520) from the comparison of the gross motor measurement data of the data (500) with the pattern function (210),
• generating comparison parameters (230) from the comparison dataset (220),
• comparing the classification parameter (520) with the comparison parameters (230),
• generating a first confidence value (540) from the comparison of the classification parameter (520) with the comparison parameters (230),
wherein the first confidence value (540) comprises a likelihood with which the user will be identified as the user registered in the communication system (100), wherein the classification result of the gross motor classification module based on the gross motor measurement data is generated from the first confidence value,
wherein the generation of the classification result (600) of the physiological state of the user is carried out only if the first confidence value (540) exceeds a threshold value.

12. The method according to claim 11, wherein the biometric data also comprise data relating to a fine motor movement of the user, wherein the plurality of physiological parameters comprise parameters of a fine motor movement, and/or
wherein the communication system (100) comprises applications, wherein the data (500) also comprise application data of the user, wherein adjustment parameters, which describe external influencing factors of the biometric data, are determined from the application data, wherein the adjustment parameters are used in the generation of the classification result (600) of the physiological state of the user to adjust the classification to the influencing factors.

13. The method according to any one of claims 11 to 12, wherein the generation of the classification result (600) of the physiological state of the user comprises a calculation of a plurality of user-specific parameters with use of the biometric data, wherein the plurality of user-specific parameters is compared with the plurality of physiological parameters of the generic states, wherein, by way of the comparison of the parameters with one another, the physiological state of the user is classified in respect of the plurality of generic states of physiological parameters.

14. The method according to any one of claims 11 to 13, wherein the communication system (100) uses a signal to signal to the user if the classification of the physiological state comprises a physiological state outside a normal range, and/or wherein the communication system (100) is configured to make an emergency call if the classification result (600) of the physiological state of the user comprises a critical classification of the physiological state of the user, and/or
wherein the plurality of generic states of the plurality of physiological parameters comprises a selection of states admissible for an authentication of the user, wherein a successful authentication of the user presupposes that the physiological state of the user is classified as an admissible state.

## Revendications

1. Système électronique de classification d'un état physiologique d'un utilisateur,
le système comprenant un système de communication (100) mobile portable, lequel comprend au moins un capteur pour la détection de données (500), un module de classification (200), un processeur (130) et une mémoire (120) interne,
dans lequel le capteur comprend un capteur interne du système de communication (100) permettant la détection de données de mesure de motricité grossière d'un déplacement du système de communication (100) occasionné par un déplacement de motricité grossière de l'utilisateur actuel du système de communication, pendant que l'utilisateur transporte l'appareil de communication avec lui,
dans lequel les données (500) comprennent des données biométriques de l'utilisateur, où les données biométriques comprennent les données de mesure de motricité grossière du déplacement de motricité grossière de l'utilisateur, où le déplacement de motricité grossière est un déplacement, lequel comprend un déplacement d'une ou des deux jambes, d'un ou des deux avant-bras, d'un ou des deux bras, du tronc, de la tête et/ou de la hanche de l'utilisateur,
dans lequel le module de classification (200) est entraîné pour la reconnaissance d'une multiplicité d'états génériques d'une multiplicité de paramètres physiologiques à l'aide d'ensembles de données d'entraînement d'une cohorte d'utilisateurs, où le module de classification (200) est exécuté par le processeur (130) du système de communication (100),
le système exécutant les étapes suivantes :
• détection des données (500) par l'au moins un capteur,
• entrée des données (500) dans le module de classification (200),
• génération d'un résultat de classification (600) de l'état physiologique de l'utilisateur à partir des données (500) par le module de classification (200),
• enregistrement du résultat de classification (600) de l'état physiologique de l'utilisateur dans la mémoire (120) du système de communication (100),
dans lequel le résultat de classification (600) de l'état physiologique de l'utilisateur comprend un profil de santé actuel de l'utilisateur,
dans lequel le profil de santé comprend une classification de l'état physiologique de l'utilisateur en ce qui concerne la multiplicité des états génériques de la multiplicité de paramètres physiologiques,
dans lequel, suite à l'entrée des données (500) dans le module de classification (200), une authentification basée sur le comportement de l'utilisateur est exécutée par un module de classification de motricité grossière du système de communication mobile portable, où le module de classification de motricité grossière est conçu pour une classification des données de mesure de motricité grossière et est entraîné pour une reconnaissance du déplacement de motricité grossière d'un utilisateur enregistré,
dans lequel au moins une fonction modèle (210) et un ensemble de données de comparaison (220) sont enregistrés dans la mémoire (120) du système de communication (100),
dans lequel l'authentification basée sur le comportement de l'utilisateur comprend :
- l'exécution répétée des étapes :
∘ détection des données de mesure de motricité grossière par le capteur interne du système de communication mobile portable,
∘ entrée des données de mesure de motricité grossière dans le module de classification de motricité grossière,
∘ génération d'un résultat de classification du module de classification de motricité grossière par le module de classification de motricité grossière si l'utilisateur est un utilisateur enregistré dans le système de communication mobile portable,
∘ enregistrement du résultat de classification du module de classification de motricité grossière dans la mémoire du système de communication mobile portable,
- suite à une demande d'authentification, accès à la mémoire du système de communication mobile portable afin de lire à partir de la mémoire au moins un des résultats de classification enregistrés du module de classification de motricité grossière,
- lecture et évaluation de l'au moins un résultat de classification du module de classification de motricité grossière selon un critère de vérification prédéfini,
- création d'un signal d'authentification dans le cas où le critère de vérification est satisfait, où le signal d'authentification signale une authentification réussie de l'utilisateur actuel,
dans lequel la génération du résultat de classification du module de classification de motricité grossière par le module de classification de motricité grossière comprend les étapes suivantes :
• comparaison des données de motricité grossière des données (500) avec l'au moins une fonction modèle (210),
• génération de paramètres de classification (520) à partir de la comparaison des données de mesure de motricité grossière des données (500) avec la fonction modèle (210),
• génération de paramètres de comparaison (230) à partir de l'ensemble de données de comparaison (220),
• comparaison des paramètres de classification (520) avec les paramètres de comparaison (230),
• génération d'une première valeur de confiance (540) à partir de la comparaison des paramètres de classification (520) avec les paramètres de comparaison (230),
dans lequel la première valeur de confiance (540) comprend une probabilité avec laquelle l'utilisateur est identifié en tant qu'utilisateur enregistré dans le système de communication (100), dans lequel, le résultat de classification reposant sur les données de mesure de motricité grossière du module de classification de motricité grossière est généré à partir de la première valeur de confiance,
dans lequel la génération du résultat de classification (600) de l'état physiologique de l'utilisateur n'est exécutée que dans le cas où la première valeur de confiance (540) dépasse une valeur de seuil.

2. Système électronique selon la revendication 1, dans lequel les données biométriques comprennent en outre un déplacement de motricité fine de l'utilisateur,
dans lequel la multiplicité de paramètres physiologiques comprend des paramètres d'un déplacement de motricité fine, et/ou*
dans lequel le système de communication (100) comprend des applications,
dans lequel les données (500) comprennent en outre des données d'application de l'utilisateur,
dans lequel des paramètres d'adaptation sont déterminés à partir des données d'application, lesquels décrivent des facteurs d'influence extérieurs des données biométriques, où les paramètres d'adaptation sont employés lors de la génération du résultat de classification (600) de l'état physiologique de l'utilisateur pour l'adaptation de la classification aux facteurs d'influence.

3. Système électronique selon l'une des revendications précédentes,
dans lequel la génération du résultat de classification (600) de l'état physiologique de l'utilisateur comprend un calcul d'une multiplicité de paramètres spécifiques à l'utilisateur moyennant l'emploi des données biométriques, où la multiplicité des paramètres spécifiques à l'utilisateur est comparée avec la multiplicité des paramètres physiologiques des états génériques, où, par la comparaison des paramètres entre eux, l'état physiologique de l'utilisateur en ce qui concerne la multiplicité des états génériques est classé par des paramètres physiologiques.

4. Système électronique selon l'une des revendications précédentes, dans lequel le système de communication (100) signale à l'utilisateur au moyen d'un signal, si la classification de l'état physiologique comprend un état physiologique en-dehors de la normale.

5. Système électronique selon l'une des revendications précédentes, dans lequel le système de communication (100) est conçu pour délivrer un appel d'urgence dans le cas où le résultat de classification (600) de l'état physiologique de l'utilisateur comprend une classification critique de l'état physiologique de l'utilisateur.

6. Système électronique selon l'une des revendications précédentes, dans lequel le système de communication (100) présente une interface, dans lequel le système de communication (100) est conçu pour communiquer avec une carte professionnelle de santé (300) électronique par le biais de l'interface, dans lequel un propriétaire d'une carte professionnelle de santé (300) électronique obtient un accès aux profils de santé de l'utilisateur enregistrés, où l'obtention de l'accès comprend les étapes suivantes :
• établissement d'une liaison sécurisée cryptographiquement entre la carte professionnelle de santé électronique et le système de communication,
• authentification du propriétaire de la carte professionnelle de santé (300),
• vérification si un état physiologique critique de l'utilisateur est présent,
• accord pour l'accès aux profils de santé de l'utilisateur enregistrés par le système de communication dans le cas où le résultat de classification (600) de l'état physiologique de l'utilisateur comprend un état physiologique critique de l'utilisateur.

7. Système électronique selon l'une des revendications précédentes, dans lequel le système de communication (100) présente un moyen de publication, où le moyen de publication indique des informations médicales concernant l'utilisateur, des informations concernant le résultat de classification (600) de l'état physiologique de l'utilisateur et/ou des mesures d'urgence pour un secouriste dans le cas où le résultat de classification (600) de l'état physiologique de l'utilisateur comprend un état physiologique critique de l'utilisateur.

8. Système électronique selon l'une des revendications précédentes, dans lequel l'authentification basée sur le comportement comprend en outre un entraînement du module de classification (200) dans la mesure où l'authentification basée sur le comportement a été réussie,
dans lequel l'entrainement du module de classification (200) comprend un ajout des données (500) à l'ensemble des données de comparaison (220) et un retrait de données (500), lesquelles sont plus anciennes qu'un âge limite, et/ou
dans lequel la multiplicité des états génériques de la multiplicité des paramètres physiologiques comprend une sélection d'états tolérés pour l'authentification, où une authentification réussie de l'utilisateur suppose que l'état physiologique de l'utilisateur est classé comme un état toléré.

9. Système électronique selon l'une des revendications précédentes, dans lequel plusieurs utilisateurs sont enregistrés sur le système de communication (100) et un résultat de classification (600) de l'état physiologique de l'utilisateur est généré pour chaque utilisateur, où un profil de santé est associé à chaque utilisateur, où le système de communication (100) présente un module de reconnaissance d'utilisateur (200), où le module de reconnaissance d'utilisateur (200) est conçu pour identifier un utilisateur en tant qu'utilisateur enregistré, où le module de reconnaissance d'utilisateur (200) est exécuté par le processeur (130) du système de communication (100),
dans lequel le module de reconnaissance de l'utilisateur est conçu pour reconnaître un changement d'utilisateur à l'aide des données (500),
dans lequel le module de reconnaissance d'utilisateur est conçu pour reconnaitre un mouvement de motricité grossière et/ou de motricité fine d'arrêt et/ou d'établissement du système de communication (100) et est entraîné pour une reconnaissance de modèles de déplacement spécifiques à l'utilisateur dans les données (500), où l'authentification basée sur le comportement comprend en outre :
• une entrée des données (500) dans le module de reconnaissable d'utilisateur,
• la génération d'au moins une deuxième valeur de confiance (540) par le module de reconnaissance d'utilisateur,
• l'évaluation de l'au moins une deuxième valeur de confiance (540) afin de vérifier s'il y a eu un échange d'utilisateur,
• l'enregistrement de l'au moins une deuxième valeur de confiance (540) dans la mémoire (120) du système de communication (100),
• l'association des données (500) à un des profils de santé de manière correspondante à la deuxième valeur de confiance (540).

10. Système électronique selon l'une des revendications précédentes, dans lequel le système de communication (100) est relié de manière communicative avec un dispositif, dans lequel le système de communication (100) crée un signal de commande, lequel est envoyé au dispositif afin de démarrer le dispositif, dans lequel le signal de commande dépend du résultat de classification (600) de l'état physiologique de l'utilisateur.

11. Procédé de classification d'un état physiologique d'un utilisateur d'un système de communication (100) mobile portable,
lequel comprend au moins un capteur permettant la détection de données (500), un module de classification (200), un processeur (130) et une mémoire (120) interne,
dans lequel le capteur comprend un capteur interne du système de communication (100) permettant la détection d'un déplacement du système de communication (100) occasionné par déplacement de motricité grossière de l'utilisateur actuel du système de communication, pendant que l'utilisateur transporte l'appareil de communication avec lui,
dans lequel les données (500) comprennent des données biométriques de l'utilisateur, où les données biométriques comprennent les données de mesure de motricité grossière du déplacement de motricité grossière de l'utilisateur, où le déplacement de motricité grossière est un déplacement, lequel comprend un déplacement d'une ou des deux jambes, d'un ou des deux avant-bras, d'un ou des deux bras, du tronc, de la tête et/ou de la hanche de l'utilisateur,
dans lequel le module de classification (200) est entraîné pour la reconnaissance d'une multiplicité d'états génériques d'une multiplicité de paramètres physiologiques à l'aide d'ensembles de données d'entraînement d'une cohorte d'utilisateurs, où le module de classification (200) est exécuté par le processeur (130) du système de communication (100),
le procédé comprenant les étapes suivantes :
• détection des données (500) par l'au moins un capteur,
• entrée des données (500) dans le module de classification (200),
• génération d'un résultat de classification (600) de l'état physiologique de l'utilisateur à partir des données (500) par le module de classification (200),
• enregistrement du résultat de classification (600) de l'état physiologique de l'utilisateur dans la mémoire (120) du système de communication (100),
dans lequel le résultat de classification (600) de l'état physiologique de l'utilisateur comprend un profil de santé actuel de l'utilisateur,
dans lequel le profil de santé comprend une classification de l'état physiologique de l'utilisateur en ce qui concerne la multiplicité des états génériques de la multiplicité de paramètres physiologiques,
dans lequel, suite à l'entrée des données (500) dans le module de classification (200), une authentification basée sur le comportement de l'utilisateur est exécutée par un module de classification de motricité grossière du système de communication mobile portable, où le module de classification de motricité grossière est conçu pour une classification des données de mesure de motricité grossière et est entraîné pour une reconnaissance du déplacement de motricité grossière d'un utilisateur enregistré,
dans lequel au moins une fonction modèle (210) et un ensemble de données de comparaison (220) sont enregistrés dans la mémoire (120) du système de communication (100),
dans lequel l'authentification basée sur le comportement de l'utilisateur comprend :
- l'exécution répétée des étapes :
∘ détection des données de mesure de motricité grossière par le capteur interne du système de communication mobile portable,
∘ entrée des données de mesure de motricité grossière dans le module de classification de motricité grossière,
∘ génération d'un résultat de classification du module de classification de motricité grossière par le module de classification de motricité grossière si l'utilisateur est un utilisateur enregistré dans le système de communication mobile portable,
∘ enregistrement du résultat de classification du module de classification de motricité grossière dans la mémoire du système de communication mobile portable,
- suite à une demande d'authentification, accès à la mémoire du système de communication mobile portable afin de lire à partir de la mémoire au moins un des résultats de classification enregistrés du module de classification de motricité grossière,
- lecture et évaluation de l'au moins un résultat de classification du module de classification de motricité grossière selon un critère de vérification prédéfini,
- création d'un signal d'authentification dans le cas où le critère de vérification est satisfait, où le signal d'authentification signale une authentification réussie de l'utilisateur actuel,
dans lequel la génération du résultat de classification du module de classification de motricité grossière par le module de classification de motricité grossière comprend les étapes suivantes :
• comparaison des données de motricité grossière des données (500) avec l'au moins une fonction modèle (210),
• génération de paramètres de classification (520) à partir de la comparaison des données de mesure de motricité grossière des données (500) avec la fonction modèle (210),
• génération de paramètres de comparaison (230) à partir de l'ensemble de données de comparaison (220),
• comparaison des paramètres de classification (520) avec les paramètres de comparaison (230),
• génération d'une première valeur de confiance (540) à partir de la comparaison des paramètres de classification (520) avec les paramètres de comparaison (230),
dans lequel la première valeur de confiance (540) comprend une probabilité avec laquelle l'utilisateur est identifié en tant qu'utilisateur enregistré dans le système de communication (100), dans lequel, le résultat de classification reposant sur les données de mesure de motricité grossière du module de classification de motricité grossière est généré à partir de la première valeur de confiance,
dans lequel la génération du résultat de classification (600) de l'état physiologique de l'utilisateur n'est exécutée que dans le cas où la première valeur de confiance (540) dépasse une valeur de seuil.

12. Procédé selon la revendication 11, dans lequel les données biométriques comprennent en outre des données d'un déplacement de motricité fine de l'utilisateur, dans lequel la multiplicité des paramètres physiologiques comprend des paramètres d'un déplacement de motricité fine, et/ou
dans lequel le système de communication (100) comprend des applications, dans lequel les données (500) comprennent en outre des données d'application de l'utilisateur, dans lequel des paramètres d'adaptation sont déterminés à partir des données d'application, lesquels décrivent des facteurs d'influence extérieurs des données biométriques, où les paramètres d'adaptation sont employés lors de la génération du résultat de classification (600) de l'état physiologique de l'utilisateur pour l'adaptation de la classification aux facteurs d'influence.

13. Procédé selon l'une des revendications 11 à 12, dans lequel la génération du résultat de classification (600) de l'état physiologique de l'utilisateur comprend un calcul d'une multiplicité de paramètres spécifiques à l'utilisateur moyennant l'emploi des données biométriques, où la multiplicité des paramètres spécifiques à l'utilisateur est comparée avec la multiplicité des paramètres physiologiques des états génériques, où, par la comparaison des paramètres entre eux, l'état physiologique de l'utilisateur en ce qui concerne la multiplicité des états génériques est classé par des paramètres physiologiques.

14. Procédé selon l'une des revendications 11 à 13, dans lequel le système de communication (100) signale à l'utilisateur au moyen d'un signal si la classification de l'état physiologique comprend un état physiologique en-dehors de la normale, et/ou dans lequel le système de communication (100) est conçu pour délivrer un appel d'urgence dans le cas où le résultat de classification (600) de l'état physiologique de l'utilisateur comprend une classification critique de l'état physiologique de l'utilisateur, et/ou
dans lequel la multiplicité des états génériques de la multiplicité de paramètres physiologiques comprend une sélection d'états tolérés pour une authentification de l'utilisateur, dans lequel une authentification réussie de l'utilisateur suppose que l'état physiologique de l'utilisateur est classifié en tant qu'état toléré.
